# EUROPEAN PATENT APPLICATION

(11) **EP 2 458 008 A1**
(43) Date of publication of application: **30.05.2012**
(21) Application number: 11194877.4
(22) Date of filing: 24.10.2006
(51) Int. Cl.: C12N 15/80

(54) **DNA binding site of a transcriptional activator useful in gene expression**

(30) Priority: 29.11.2005 EP 05111442
(62) Divisional of application: 06807517.5
(71) Applicant: DSM IP Assets B.V., 6411 TE Heerlen (NL)
(72) Inventor: Parenicova, Lucie, 2497 ZJ Den Haag (NL); Peij, Van, Noël Nicolaas Maria Elisabeth, 2645 KJ Delfgauw (NL)
(74) Representative: Monaco, Vania

(57) **Abstract**

We have discovered DNA binding sites which are specifically recognized by PrtT, a transcriptional activator for protease genes. The DNA binding site can be defined structurally by a consensus nucleotide sequence and functionally by PrtT's ability to regulate transcriptional activation through that sequence. Both PrtT and its cognate DNA binding site (*i.e*., the nucleotide sequence in each promoter that is recognized by PrtT) can be used in a gene expression system. Possession of only a PrtT transcriptional activator is insufficient, its cognate DNA binding site is necessary for recognition by PrtT (*i.e*., binding to the site and activating transcription under appropriate conditions). A functional site, such as one obtained from a wild-type fungal gene, will confer PrtT-dependent transcriptional activation on 3'-downstream sequences. A mutation of a wild-type promoter that results in a non-functional site will abolish PrtT-dependent transcriptional activation of 3'-downstream sequences. A mutation of a wild-type promoter that results in a more functional site will enhance PrtT-dependent transcriptional activation of 3'-downstream sequences.

## Description

### Field of the Invention

The present invention relates to polynucleotides having a functional, a mutated, non-functional and/or a mutated, enhanced DNA binding site which is specifically recognized by PrtT, a transcriptional activator for protease genes, and their use in regulating gene expression.

### Background of the Invention

Fungal transcriptional activators named PrtT have been recently described in WO 00/20596, WO 01/68864 and WO 06/040312. These transcriptional activators were isolated from *Aspergillus niger (A. niger)* and *Aspergillus oryzae (A. oryzae*)*.* They globally activate transcription from the 5'-upstream promoters of fungal protease genes. Until recently, modulation of PrtT activated transcription was only possible on a global level by altering the transcriptional activator itself.

We now present conserved DNA binding sites in promoters of protease genes that are necessary and sufficient to confer PrtT-dependent transcriptional activation on a downstream nucleotide sequence.

The present invention provides a novel DNA binding site located in a promoter region and recognized by PrtT transcriptional activators, which has improved properties for regulating transcription of genes in fungi as compared to sequences that have been previously described. Other advantages and improvements are described below or would be apparent from the disclosure herein.

### Summary of the Invention

It is an object of the invention to control, by genetic engineering, PrtT responsiveness, enhanced responsiveness or non-responsiveness of a native fungal gene or other heterologous nucleotide sequence through recognition or non-recognition of the promoter's functional or mutated DNA binding site in fungal cells.

In a first aspect of the invention, polynucleotides are provided that comprise one or more DNA binding sites, wherein at least one site is recognized by PrtT and confers PrtT-dependent transcriptional activation.

In a second aspect of the invention, polynucleotides are provided that comprise one or more mutated, non-functional DNA binding sites, wherein at least one site is not recognized by PrtT and/or does not confer PrtT-dependent transcriptional activation.

In a third aspect of the invention, polynucleotides are provided that comprise one or more mutated, enhanced DNA binding sites, wherein at least one site is recognized by PrtT and confers enhanced PrtT-dependent transcriptional activation.

In a fourth aspect of the invention, expression vectors comprising at least one of either of the aforementioned non-mutated and /or mutated, enhanced DNA binding sites are provided. The vector may further comprise a downstream nucleotide sequence which is transcribed by the promoter which contains a functional or functional, enhanced DNA binding site and is activated by PrtT. In addition, cells comprising an aforementioned DNA binding site (e.g. non-mutated, mutated, non-functional and mutated, enhanced) and/or an aforementioned vector are provided.

In a fifth aspect of the invention, cells in which at least one endogenous gene is mutated such that its promoter is no longer bound or transcriptionally activated by PrtT, or cells comprising the mutated, non-functional aforementioned polynucleotides or respective expression vector are provided. Such cells with at least reduced protease activity are also provided.

In a sixth aspect of the invention, processes for identifying protease genes are provided. One or more differentially expressed genes are detected between fungal cells either with or without a PrtT transcriptional activator (*e.g*., deleting *prt*T or otherwise inactivating PrtT). Those differentially expressed genes that encode proteases (*e.g.,* as determined by sequence similarity to known proteases, proteolytic activity of the gene product, and possession of a DNA binding site for PrtT) are identified as protease genes controlled by PrtT. Novel proteases so identified and polynucleotides encoding them are also provided.

In a seventh aspect of the invention, processes for producing a cell with at least one mutated, non-functional DNA binding site in a promoter are provided. The mutation may be introduced into a host chromosome (*i.e*., an endogenous gene, preferably a protease gene) by mutagenesis or recombination techniques. Introduction of the mutation into the host genome may be confirmed by reduced binding of PrtT to the mutated DNA binding site or reduced PrtT-dependent transcriptional activation from the promoter. Inactivation of the endogenous promoters of at least 13 experimentally identified, more preferably all native protease genes by mutating a like number of DNA binding sites is preferred.

In an eighth aspect of the invention, processes for producing a polypeptide are provided. One or more polypeptides are expressed in a cell from an expression vector or an endogenous promoter of a native fungal gene. For protease-sensitive polypeptides, the cell is preferably reduced in protease activity. For polypeptides (such as many proteases) that are secreted out of the cell, they may be recovered from the nutrient medium. Otherwise, polypeptides are recovered from the cells (preferably a cell paste or pellet): (i) soluble polypeptides from a cell lysate and (ii) insoluble polypeptides or those inserted into or associated with cell membranes from a cell fraction.

Other processes for using and making the aforementioned polynucleotides, expression vectors, novel proteases and the polynucleotides encoding them, and cells are also provided. Further aspects of the invention will be apparent from the following description and claims, and generalizations thereto.

### Brief Description of the Drawings and Tables

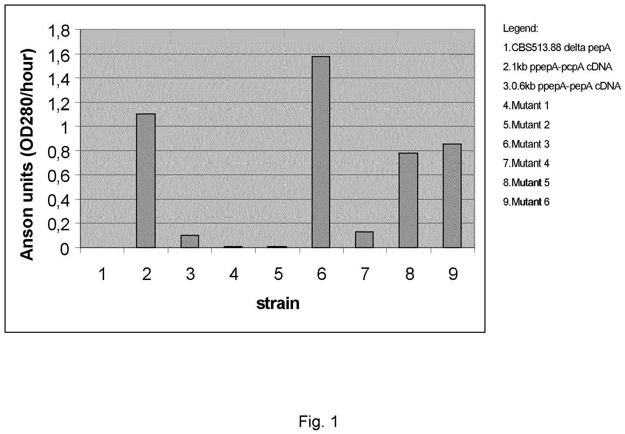

Figure 1 shows the measurement of protease activity in culture supernatants. Results of the Anson assay (J. Gen. Physiol. 22:79-89, 1938) were determined using supernatant after 10 days fermentation in a shaker flask. Protease activity measurements in CBS 513.88 delta *pep*A transformants carrying constructs with differently modified *pep*A promoters (see Table 5) are shown in lanes 4 to 9. The lanes 2 and 3 correspond to transformants carrying the wild type 1.0kb *pep*A promoter (containing the PrtT binding site) and the wild type 0.6kb *pep*A promoter (without the PrtT binding site) constructs, respectively. The background was determined as the protease activity of non-transformed *Aspergillus niger* CBS 513.88 delta pepA (lane 1). Each bar represents a mean value of protease activity of two independent transformants.

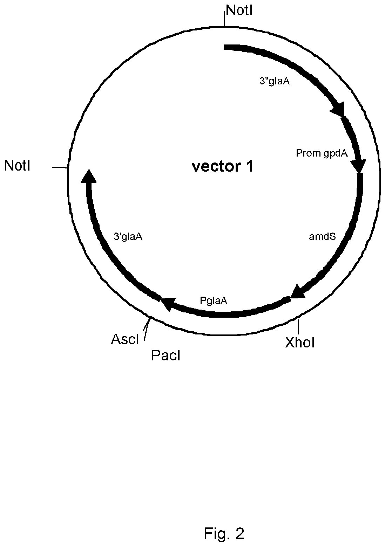

Figure 2 shows a physical plasmid map of vector 1, which illustrates recognition sites for restriction enzymes, the locations of genes, and their orientation. It is used for recloning *Asc*I/*Xho*I p*pep*A*-pep*A constructs.

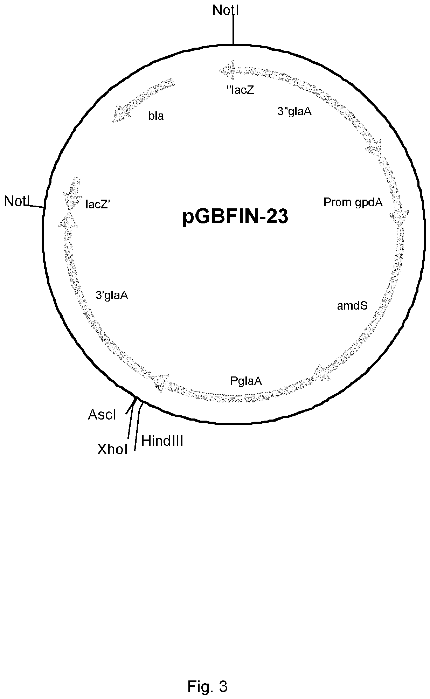

Figure 3 shows a physical plasmid map of the expression vector pGBFIN-23, which illustrates recognition sites for restriction enzymes, the locations of genes, and their orientation. Indicated are the *gla*A flanking regions relative to the *gla*A promoter and the *Hind*III*-Xho*I cloning site. The *E. coli* DNA can be removed by digestion with restriction enzyme *Not*l prior to transformation of an *A. niger* strain.

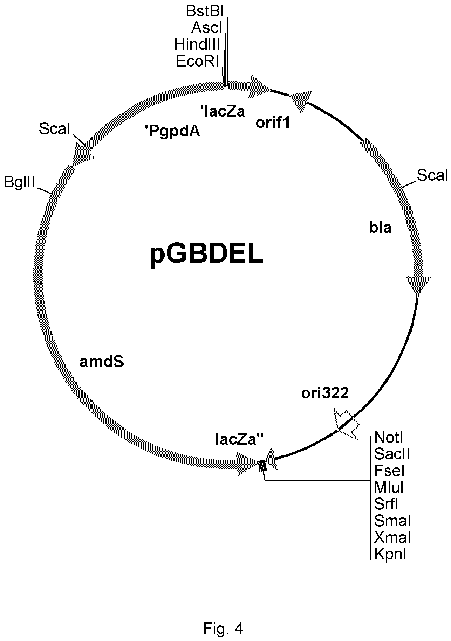

Figure 4 shows a physical plasmid map of the replacement vector pGBDEL, which illustrates recognition sites for restriction enzymes, the locations of genes, and their orientation. Indicated are the multiple cloning sites for cloning the flanking regions relative to the *amd*S marker.

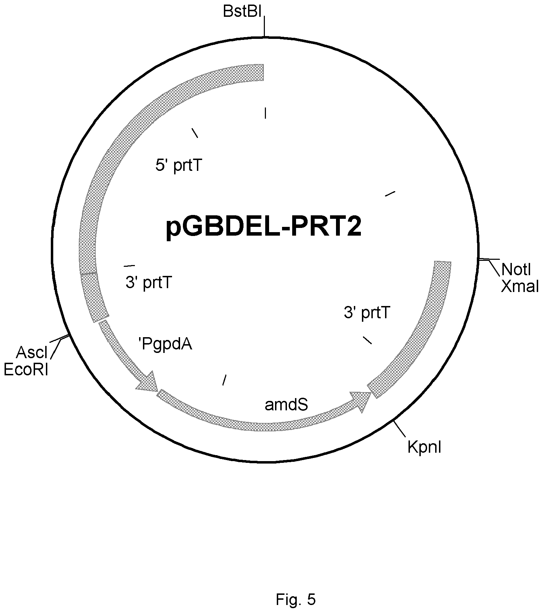

Figure 5 shows a physical plasmid map of the replacement vector pGBDEL-PRT2, which illustrates recognition sites for restriction enzymes, the locations of genes, and their orientation. Indicated are the 5' *prt*T flanking region and the 3' *prt*T flanking regions relative to the *amd*S marker. The sequences of the 3' *prt*T flanking regions overlap by at least a few hundred basepairs. *E. coli* DNA was removed by digestion with restriction enzymes *Bst*BI and *Xma*I, and subsequent recircularization prior to transformation of an *A*. *niger* strain.

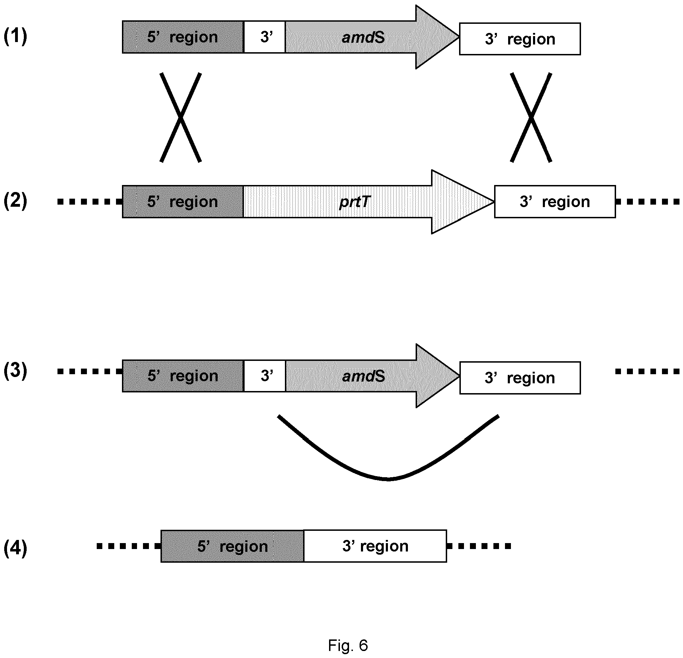

Figure 6 illustrates a procedure for deletion of the chromosomal *prt*T gene. A linear DNA construct of pGBDEL-PRT2 comprising the *amd*S selection marker flanked by homologous regions (5' and 3') of the *prtT* gene (1), integrates through double-crossover homologous recombination (X) in the genome at the *prtT* genetic locus (2), and replaces the chromosomal *prtT* gene (3). Subsequently, recombination between the direct repeats (3', 3' region) removes the *amd*S marker and results in precise excision of the *prtT* gene (4).

### Detailed description of the Invention

The DNA binding site can be defined structurally by a consensus nucleotide sequence and functionally by PrtT's ability to regulate transcriptional activation through that sequence. The DNA binding site was identified as a conserved nucleotide sequence (SEQ ID NO: 22 is the extended sequence) in endogenous promoters of native fungal protease genes. Both PrtT and its cognate DNA binding site (*i.e*., the nucleotide sequence in each promoter that is recognized by PrtT and confers to the gene ability to be transcriptionally controlled by PrtT) can be used in a gene expression system either (i) to improve a method for producing polypeptides in a PrtT-containing cell or (ii) to improve a method for producing a protease-sensitive polypeptide in a PrtT-containing cell. Possession of only a PrtT transcriptional activator is insufficient, because locating its cognate DNA binding site in the promoter is necessary for recognition by PrtT (*i.e*., binding to the site and activating transcription from the promoter under appropriate conditions). A functional site, such as one obtained from a wild-type fungal protease gene, will confer PrtT-dependent transcriptional activation on 3'-downstream sequences. Conversely, mutation of a wild-type promoter that results in a non-functional site will abolish PrtT-dependent transcriptional activation of 3'-downstream sequences. In the sequence listing of the present invention SEQ ID NO: 22 depicts the PrtT DNA binding site as [snnnnnccgw cggnnnnnnn nnnnnnnnnn nns]. For clarity reasons, in the description of the present invention SEQ ID NO: 22 will be depicted as 5'-G/C (N)₅ C C G A/T C G G (N)₁₉ G/C-3', i.e. G/C for S, and A/T for W.

In an embodiment of the present invention, a polynucleotide is provided that comprises one or more double-stranded DNA binding sites. At least 32 bases of a first strand of a site are identical in sequence to 5'-G/C (N)₅ C C G A/T C G G (N)₁₉ G/C-3' (SEQ ID NO:22). Since 24 bases in the extended DNA binding site may be degenerate, there can be changes in the sequence at only nine positions to alter binding affinity of PrtT and/or to alter PrtT activated transcription of a downstream nucleotide sequence. A second strand of the site is complementary to the first strand. The site is specifically bound by a PrtT transcriptional activator under binding conditions and such binding will activate transcription of a downstream nucleotide sequence. The asymmetry of the extended sequence above or the shorter 5'-C C G A/T C G G-3' is helpful in defining the direction of transcription as being in the 5' → 3' direction. It is preferred that a functional DNA binding site conserve the imperfect palindromic sequence of seven contiguous bases. The double-stranded DNA binding site may be obtained from a eukaryote (*e.g.*, fungus).

In another embodiment of the present invention, a polynucleotide is provided comprising one or more double-stranded mutated, non-functional DNA binding sites. A double-stranded DNA binding site (*e.g*., at least 32 bases of a first strand of the non-mutated site are identical in sequence to 5'-G/C (N)₅ C C G A/T C G G (N)₁₉ G/C-3', SEQ ID NO:22) is obtained from a eukaryote (*e.g.*, fungus), wherein the non-mutated, functional site is specifically bound by a PrtT transcriptional activator under binding conditions and such binding will activate transcription of a downstream nucleotide sequence. But at least one (*e.g.*, one, two, three, four, five, six, seven, eight, or nine) bases of a first strand of the mutated site are changed as compared to the sequence of the non-mutated site (*e.g*., by addition, deletion, transition, transversion, or any combination thereof of bases in the extended sequence) such that PrtT no longer specifically recognizes the mutated, non-functional site or that PrtT no longer activates transcription. Since 24 bases in the extended DNA binding site may be degenerate, there can be changes in the sequence at a maximum of nine positions to alter binding affinity of PrtT and/or to alter PrtT activated transcription of a downstream nucleotide sequence. A second strand of the mutated, non-functional site is complementary to the first strand. The non-mutated site was specifically bound by a PrtT transcriptional activator under binding conditions and such binding would have activated transcription downstream. It is preferred that a mutated, non-functional DNA binding site has one or more changes in the imperfect palindromic sequence of seven contiguous bases.

In yet another embodiment of the present invention, a polynucleotide is provided that comprises one or more double-stranded mutated, enhanced DNA binding sites that demonstrate enhanced transcription of a downstream nucleotide sequence as compared to the non-mutated DNA binding site. A double-stranded DNA binding site (e.g., at least 32 bases of a first strand of the non-mutated site are identical in sequence to 5'-G/C (N)₅ C C G A/T C G G (N)₁₉ G/C-3', SEQ ID NO:22) is obtained from a eukaryote (*e.g.*, fungus), wherein the non-mutated and mutated, enhanced sites are specifically bound by a PrtT transcriptional activator under binding conditions and such binding will activate transcription of a downstream nucleotide sequence. But at least one (*e.g*., one, two, three, four, five, six, seven, eight, or nine) bases of a first strand of the mutated site are changed as compared to the sequence of the non-mutated site (*e.g*., by addition, deletion, transition, transversion, or any combination thereof of bases in the extended sequence) such that PrtT specifically recognizes the site and activates and enhances transcription of the downstream nucleotide sequence. Since 24 bases in the extended DNA binding site may be degenerate, there can be changes in the sequence at a maximum of nine positions to alter binding affinity of PrtT and/or to alter PrtT activated transcription of a downstream nucleotide sequence. A second strand of the mutated site is complementary to the first strand. The non-mutated and mutated, enhanced sites are specifically bound by a PrtT transcriptional activator under binding conditions and such binding would have activated transcription downstream. It is preferred that a mutated, enhanced DNA binding site has one or more changes in both the imperfect palindrome sequence of seven contiguous bases and in the extended sequence.

The term "enhanced transcription of a nucleotide sequence downstream of a mutated DNA binding site" in the context of the present invention is defined as at least 10% more, at least 20% more, at least 30% more, at least 40% more, at least 80% more, at least 100% more, at least 200% more, or at least 300% more transcription product than a corresponding nucleotide sequence downstream of a non-mutated DNA binding site when expressed under the same conditions using the same assay. Assays to monitor the amount of expression product are known to the person skilled in the art. Examples of such assays to monitor the amount of expression product are: Northern Blot, Quantititive Polymerase Chain Reaction (PCR), Real Time PCR, Gene Array analysis.

A polynucleotide may be isolated from a eukaryotic source (e.g., fungus). For example, it may be cloned by screening a source library of recombinant sequences or amplifying a fragmentary sequence from source DNA. Thus, it may be purified to any desired degree from the eukaryote (e.g., at least 90% of solutes in a composition are the desired nucleic acid molecules). A promoter sequence from a non-fungal source that does not possess a PrtT transcriptional activator may fortuitously be identical to SEQ ID NO:22 or similar enough to be corrected to SEQ ID NO:22 and, therefore, regulated by PrtT in a cell. It may be recombinant if DNA which is not colinear in the genome are joined together to construct a recombinant polynucleotide. At least some parts of the polynucleotide are likely to have been obtained from sources other than the cell in which the polynucleotide is replicated.

In an expression vector, the base contacts, orientation, and spacing of the one or more DNA binding sites is typical for a zinc finger-containing transcription factor such as PrtT. The DNA binding site may be inserted into the expression vector for optimal or suboptimal transcriptional activation of a downstream nucleotide sequence (*e.g.,* protease gene or other gene, either native or heterologous) located within 1200 bases, 800 bases, or between 100 and 1200 bases of the site of transcriptional initiation. The downstream nucleotide sequence (e.g. coding sequence) may be a non-protease gene, a non-fungal gene, or both. Other control sequences of the promoter may or may not be derived from a fungal gene.

Two different types of PrtT-expressing fungal cells may be engineered using the above embodiments of the invention. Fungal cells are engineered by inserting functional DNA binding sites into the chromosome or on an episome to confer PrtT-dependent transcriptional activation on a nucleotide sequence downstream of the site. This type of fungus will coordinately transcribe such sequences with protease genes. Alternatively, fungal cells are engineered by replacing chromosomal or episomal DNA binding sites with mutated, non-functional sites to abolish PrtT-responsiveness because a promoter comprising the mutated, non-functional site is no longer recognized by PrtT. Native protease genes may be inactivated in this manner; PrtT-dependent transcriptional activation of a nucleotide sequence downstream of a functional site may also be inactivated. This type of fungus is suitable for expressing native or heterologous polypeptides that are sensitive to protease degradation. Thus, the polynucleotide of the invention may be a recombination vector comprising a functional or non-functional DNA binding site flanked by homologous sequences of the target locus and a selectable marker to detect recombination, but does not require the presence of a downstream coding sequence for expression by the promoter. Preferably the homologous flanking sequences comprise at least 30 bases, more preferably at least 50 bases, more preferably at least 100 bases, at least 200 bases, more preferably at least 500 bases, even more preferably at least 1 kb, most preferably at least 2 kb of the target locus of the host cell. By rendering a PrtT binding site non-functional, specific proteases can be inactivated, depending on the protein of interest to be produced (e.g. inactivation of proline specific proteases when the protein of interest is rich in prolines, or inactivate carboxypeptidases when the protein of interest is sensitive to degradation by carboxypeptidases). In contrast, global inactivation of proteases by deletion of the PrtT gene has the disadvantage that those proteases necessary for intracellular processes and protein regulation will be inactivated as well, leading to an impaired expression host cell. Furthermore, other genes than proteases (e.g. genes encoding transporters) controlled by PrtT can be essential for the cell, and inactivation of PrtT may be lethal for those cells.

PrtT polypeptides are transcriptional activators of protease genes acting at DNA binding sites in their promoters. The term "transcriptional activator" as used herein refers to a polypeptide which has the ability to activate transcription from amongst others: a specific protease promoter or a set of protease promoters. PrtT is necessary for the initiation of transcription of the protease gene (or other heterologous nucleotide sequence) to which the promoter is operably linked.

The biological activity of a DNA binding site which is contained in a protease promoter may be determined by measuring the protease's activity (*e.g*., proteolysis) as described herein for determination of acidic endo-protease activity using bovine serum albumin (BSA) as the substrate. This method is also described by van den Hombergh et al. (Curr. Genet. 28:299-308, 1995). Other methods for measuring a protease's activity can be found in WO 02/068623. Alternatively, the biological activity of the DNA binding site can be determined by measuring the mRNA level of the protease transcripts. The mRNA levels can, for example, be quantitated by hybridization (*e.g.,* Northern or slot blotting) or RT-PCR. A filter binding assay, protein crosslinking, and chromatin immunoprecipitation may be used to identify polypeptide binding to the DNA binding site.

A reporter gene under the control of a promoter comprising the DNA binding site can be used as a surrogate for the protease or its activity. Measuring the biological activity of β-galactosidase (lacZ) or green fluorescent protein (GFP) reporter has been described (Luo, Gene 163:127-131, 1995; Henriksen et al., Microbiol. 145:729-734, 1999). Alternatively, one or more specific protease reporter genes such as the pepstatin-sensitive extracellular aspartic protease encoding *pep*A gene can be used for measuring the activity of a DNA binding site. One or more sites regulate transcription of the reporter gene's promoter.

At least one mutation may be introduced in a DNA binding site by standard techniques, such as site-directed mutagenesis and PCR-mediated mutagenesis. Example of mutagenesis procedures are the QuickChange™ site-directed mutagenesis kit (Stratagene), the Altered Sites^{®} II *in vitro* mutagenesis system (Promega), sequence overlap extension (SOE-PCR as described by Ho et al., Gene 77:51-59, 1989), or other PCR techniques (Griffin & Griffin, eds., Molecular Biology: Current Innovations and Future Trends, Horizon Scientific Press, Norfolk, UK). Random mutagenesis (*e.g.,* chemical or radiation damage) or error-prone DNA replication (*e.g.*, nucleotide misincorporation) may also be used to introduce mutations in the DNA binding site.

The DNA binding site of the present invention may be obtained from any filamentous fungus. "Filamentous fungi" include all filamentous forms of the subdivision Eumycota and Oomycota. The filamentous fungi are characterized by a mycelia wall composed of chitin, cellulose, glucan, chitosan, mannan, and other complex polysaccharides. Vegetative growth is by hyphal elongation and carbon catabolism is obligately aerobic. Filamentous fungal species include, but are not limited to, those of the genus *Acremonium, Aspergillus, Aureobasidium, Cryptococcus,Filibasidium, Fusarium, Humicola, Magnaporthe, Mucor, Myceliophthora, Neocallimastix, Neurospora, Paecilomyces, Penicillium, Piromyces, Schizophyllum, Talaromyces,Thermoascus, Thielavia, Tolypocladium,* and *Trichoderma.*

The DNA binding site may be obtained from a species of *Aspergillus,* such as A. *awamori, A. nidulans, A. niger, A. oryzae, A. sojae,* or *A*. *fumigatus.* Preferably, it is obtained from a strain of *A*. *niger, A. oryzae,* or *A*. *fumigatus.* Alternatively, it is obtained from a species of *Penicillium,* such as *P. chrysogenum,* or a species of *Fusarium,* such as *F. oxysporum* or *F. venenatum.*

It will be understood that for the aforementioned species, the invention encompasses both the perfect and imperfect states, and other taxonomic equivalents, e.g., anamorphs, regardless of the species name by which they are known. Those skilled in the art will readily recognize the identity of appropriate equivalents. For example, the polypeptides may be obtained from microorganisms, which are taxonomic equivalents of *Aspergillus* as defined by Raper & Fennel (The Genus Aspergillus, Wilkins Company, Baltimore MD, 1965) regardless of the species name by which they are known.

*Aspergilli* are mitosporic fungi characterized by an aspergillum comprising a conidiospore stipe with no known teleomorphic states terminating in a vesicle, which in turn bears one or two layers of synchronously formed specialized cells, variously referred to as sterigmata or phialides, and asexually formed spores referred to as conidia. Known teleomorphs of *Aspergillus* include *Eurotium, Neosartorya,* and *Emericella.* Strains of *Aspergillus* and teleomorphs thereof are readily accessible to the public in a number of culture collections.

DNA binding sites may be obtained from strains of filamentous fungus such as for example *Aspergillus niger* CBS 513.88, *Aspergillus oryzae* ATCC 20423, IFO 4177, ATCC 1011, ATCC 9576, ATCC14488-14491, ATCC 11601, ATCC12892, *Aspergillus fumigatus* AF293 (CBS101355), *P. chrysogenum* CBS 455.95, *Penicillium citrinum* ATCC 38065, *Penicillium chrysogenum* P2, *Acremonium chrysogenum* ATCC 36225 or ATCC 48272, *Trichoderma reesei* ATCC 26921 or ATCC 56765 or ATCC 26921, *Aspergillus sojae* ATCC11906, *Chrysosporium lucknowense* ATCC44006, and derivatives thereof.

Furthermore, DNA binding sites may be identified and obtained from other sources including microorganisms isolated from nature (*e.g*., soil, composts, water, etc.). Techniques for isolating microorganisms from the environment are known in the art. The nucleotide sequence may be derived with a labeled probe by screening a genomic library of another microorganism. Once a nucleotide sequence of at least one DNA binding site has been detected with the probe, the sequence may be isolated or cloned by utilizing known techniques (see, *e.g*., Sambrook & Russell, Molecular Cloning: A Laboratory Manual, 3rd Ed., CSHL Press, Cold Spring Harbor, NY, 2001; and Ausubel et al., Current Protocols in Molecular Biology, Wiley InterScience, NY, 1995).

Low- to medium- to high-stringency conditions means prehybridization and hybridization at 42°C in 5x SSPE, 0.3% SDS, 200 pg/ml sheared and denatured salmon sperm DNA, and one of 25%, 35% or 50% formamide for low to medium to high stringencies, respectively. Subsequently, the hybridization reaction is washed three times for 30 min each using 2x SSC, 0.2% SDS and one of 55°C, 65°C or 75°C for low to medium to high stringencies, respectively. Oligonucleotide probes may be used. They are typically labeled for detecting corresponding DNA binding sites (*e.g*., with ³²P, ³³P, ³H, ³⁵S, biotin, avidin, or a fluorescent/luminescent marker). For example, molecules to which a ³²P-, ³³P-, ³H- or ³⁵S-labeled oligonucleotide probe hybridizes may be detected by use of X-ray film or Phospho-Image™ analysis.

A variant of the nucleotide sequence may also be a homologous or paralogous DNA binding site in a protease promoter. In the context of the invention, homologous or paralogous means nucleotide sequence identical or similar to 5'-C C G A/T C G G-3' and obtained from *A. niger, A. oryzae, A. fumigatus, P. chrysogenum, F. oxysporum,* or *F. venenatum.* For example, *Aspergillus* strains can be screened for a homologous or paralogous DNA binding site in a protease promoter by hybridization. Upon detection of a homologous or paralogous nucleotide sequence according to the present invention, a genomic DNA library can be screened using a probe that hybridizes to 5'-C C G A/T C G G-3' or more extended versions thereof, e.g. the extend versions as depicted Table 2 and 3.

The techniques used to isolate or clone a nucleotide sequence are known in the art and include direct isolation from genomic DNA. The cloning of a nucleotide sequence of the present invention from such genomic DNA can be accomplished by using known hybridization techniques.

"Polynucleotide" is defined herein as a double-stranded nucleic acid molecule, which is isolated from a naturally occurring gene or which has been modified to contain segments of nucleic acid which are combined and juxtaposed in a manner which would not otherwise exist in nature. "Expression vector" is defined as a double-stranded nucleic acid molecule, which contains control sequences required for expression of a coding sequence and maintenance (at least temporarily) in a cell. The term "coding sequence" as defined herein is a sequence, which is transcribed into mRNA and translated into a polypeptide. The boundaries of the coding sequence are generally determined by the ATG start codon at the 5' end of the mRNA and a translation stop codon sequence terminating the open reading frame at the 3' end of the mRNA. A coding sequence can include, but is not limited to, DNA, cDNA, and recombinant nucleotide sequences. Expression will be understood to include any step involved in the production of the polypeptide including, but not limited to, transcription, splicing, posttranscriptional modification, translation, posttranslational modification, secretion, and proteolytic processing.

The term "control sequences" is defined herein to include all components, which are necessary or advantageous for the expression of a polypeptide. Each control sequence may be native or foreign to the nucleotide sequence encoding the polypeptide. Such control sequences include, but are not limited to, a leader, optimal translation initiation sequences (as described in Kozak, J. Biol. Chem. 266:19867-19870, 1991), polyadenylation sequence, propeptide sequence, prepropeptide sequence, promoter, signal sequence, and transcription terminator. At a minimum, the control sequences include a promoter, and transcriptional and translational stop signals.

Manipulation of the nucleotide sequence encoding a polypeptide prior to its insertion into an expression vector may be desirable or necessary depending on the vector. The techniques for modifying nucleotide sequences utilizing cloning methods are known in the art. The control sequences may be provided with linkers for the purpose of introducing specific restriction sites facilitating ligation of the control sequences with the coding region of the nucleotide sequence encoding a polypeptide. The term "operably linked" is defined herein as a configuration in which a control sequence is appropriately placed at a position relative to the coding sequence such that the control sequence directs expression of a polypeptide or transcription of other downstream sequences.

The control sequence may be a promoter, which is recognized by the cellular machinery for expression of a downstream nucleotide sequence. The promoter contains transcriptional control sequences (*e.g*., one or more DNA binding sites of the present invention) that regulate the expression of the polypeptide or transcription of other downstream sequences. The promoter may be any nucleotide sequence, which shows transcriptional activity in the cell including mutant, truncated, and hybrid promoters, and may be obtained from genes encoding extracellular or intracellular polypeptides either native or heterologous to the cell.

The control sequence may also be a suitable transcription terminator sequence, a nucleotide sequence recognized by the cellular machinery to terminate transcription. The terminator sequence is operably linked to the 3' terminus of the nucleotide sequence encoding the polypeptide. Any terminator, which is functional in the cell, may be used in the present invention. Preferred terminators for filamentous fungal cells are obtained from the genes encoding *A. oryzae* TAKA amylase, *A. niger* glucoamylase (*glaA*), *A*. *nidulans* anthranilate synthase, *A*. *niger* alpha-glucosidase, *trp*C gene, and *Fusarium oxysporum* trypsin-like protease.

The control sequence may also be a suitable leader sequence, a non-translated region of a mRNA which is important for translation by the cell. The leader sequence is operably linked to the 5' terminus of the nucleotide sequence encoding the polypeptide. Any leader sequence, which is functional in the cell, may be used in the present invention. Preferred leaders for filamentous fungal cells are obtained from the genes encoding *A*. *oryzae* TAKA amylase and *A*. *nidulans* triose phosphate isomerase and *A*. *niger glaA.* Other control sequences may be isolated from the *Penicillium* I PNS gene, or *pcb*C gene, the beta tubulin gene. All of the control sequences cited in WO 01/21779 are herewith incorporated by reference.

The control sequence may also be a polyadenylation sequence, a sequence which is operably linked to the 3' terminus of the nucleotide sequence and which, when transcribed, is recognized by the filamentous fungal cell as a signal to add polyadenosine residues to transcribed mRNA. Any polyadenylation sequence, which is functional in the cell, may be used in the present invention. Preferred polyadenylation sequences for filamentous fungal cells are obtained from the genes encoding *A. oryzae* TAKA amylase, *A. niger* glucoamylase, *A. nidulans* anthranilate synthase, *Fusarium oxysporum* trypsin-like protease, and *A*. *niger* alpha-glucosidase.

### Expression Vectors

Yet another embodiment of the present invention are expression vectors comprising a DNA binding site within the promoter, a downstream transcribed sequence, a transcriptional stop signal, a translational stop signal, and other control sequences for maintenance of the vector in a cell produced by recombinant technology. The various nucleotide and control sequences described above may be joined together to produce a recombinant expression vector which may include one or more convenient restriction sites to allow for insertion or substitution of the nucleotide sequence encoding the polypeptide at such sites.

A nucleotide sequence encoding a desired polypeptide may be expressed by inserting the nucleotide sequence or a polynucleotide comprising the nucleotide sequence into an appropriate vector for expression. In creating the expression vector, the coding sequence is located in the vector so that the coding sequence is operably linked with the appropriate control sequences (*e.g.,* one or more DNA binding sites) for expression, and possibly secretion.

The expression vector may be any vector (*e.g*., a plasmid or virus), which can be conveniently engineered by recombinant technology and can bring about the expression of the nucleotide sequence encoding a desired polypeptide. The choice of the vector will typically depend on the compatibility of the vector with the cell (*e.g.*, filamentous fungus) into which the vector is to be introduced. The vectors may be linear or a closed circle (*i.e*., episome). The vector may be an autonomously replicating vector, *i.e.*, a vector, which exists as an extrachromosomal entity, the replication of which is independent of chromosomal replication, *e.g.,* plasmid, extrachromosomal element, minichromosome, or artificial chromosome. An autonomously maintained cloning vector may comprise the AMA1-sequence (see, *e.g.*, Aleksenko & Clutterbuck, Fungal Genet. Biol. 21:373-397, 1997). Positive and negative selectable markers in the vector may be used for genetic engineering. Maintenance functions and an origin of replication are essential for episomes. Engineered chromosomes may require a centromere, telomeres, and origins of replication for maintenance and segregation.

Alternatively, the vector may be one which, when introduced into the cell (*e.g*., a filamentous fungus), is integrated into the genome and replicated together with the chromosome into which it has been integrated. The integrative cloning vector may integrate at random or at a predetermined target locus in the chromosome of the cell. In an embodiment of the present invention, the integrative cloning vector comprises a fragmentary region, which is homologous to a nucleotide sequence at a predetermined target locus in the genome of the cell for targeting integration of the cloning vector at the locus. In order to promote targeted integration, the cloning vector is preferably linearized prior to transformation of the cell. Linearization is preferably performed such that at least one but preferably either end of the cloning vector is flanked by sequences homologous to the target locus to allow integration by homologous recombination. The skilled person will know the optimal length of a flanking sequence for a specific host cell to allow integration by homologous recombination. The length of the homologous sequences flanking the target locus is preferably at least 30 bp, preferably at least 50 bp, preferably at least 0.1 kb, even preferably at least 0.2 kb, more preferably at least 0.5 kb, even more preferably at least 1 kb, most preferably at least 2 kb. Preferably, the efficiency of targeted integration into the genome of the host cell, i.e. integration in a predetermined target locus, is increased by augmented homologous recombination abilities of the host cell. Such phenotype of the cell preferably involves a deficient ku70 gene as described in WO2005/095624. WO2005/095624, which is herein enclosed by reference, discloses a preferred method to obtain a filamentous fungal cell comprising increased efficiency of targeted integration. Preferably, a DNA sequence in the cloning vector, which is homologous to the target locus is derived from a highly expressed locus meaning that it is derived from a gene, which is capable of high expression level in the filamentous fungal host cell. A gene capable of high expression level, *i.e.* a highly expressed gene, is herein defined as a gene whose mRNA can make up at least 0.5% (w/w) of the total cellular mRNA, *e.g*. under induced conditions, or alternatively, a gene whose gene product can make up at least 1% (w/w) of the total cellular protein, or, in case of a secreted gene product, can be secreted to a level of at least 0.1 g/l (as described in EP 357127). A number of preferred highly expressed fungal genes are given by way of example: the amylase, glucoamylase, alcohol dehydrogenase, xylanase, glyceraldehyde-phosphate dehydrogenase or cellobiohydrolase (*cbh*) genes from *Aspergilli* or *Trichoderma.* Most preferred highly expressed genes for these purposes are a glucoamylase gene, preferably an *A*. *niger* glucoamylase gene, an *A*. *oryzae* TAKA-amylase gene, an *A*. *nidulans gpdA* gene, a *Trichoderma reesei cbh* gene, preferably *cbh*1*.* They may be fungal genes regulated by the DNA binding sites of the present invention. More than one copy of a nucleotide sequence encoding a polypeptide may be inserted into the cell to increase production of the gene product. This can be done, preferably by integrating into the cell's chromosome of the nucleotide sequence, more preferably by targeting the integration of the nucleotide sequence at one of the highly expressed loci listed immediately above. Integration may be enhanced by a recombinase. Alternatively, this can be done by including an amplifiable selectable marker gene with the nucleotide sequence where cells containing amplified copies of the selectable marker gene, and thereby additional copies of the nucleotide sequence, can be selected for by cultivating the cells in the presence of the appropriate selectable agent. To increase even more the number of copies of the DNA sequence to be over expressed, the technique of gene conversion as described in WO 98/46772 may be used.

The vectors preferably contain one or more selectable markers, which permit easy selection of transformed cells. A selectable marker is a gene the product of which provides for biocide or viral resistance, resistance to heavy metals, prototrophy to auxotrophs, and the like. A selectable marker for use in a filamentous fungal cell may be selected from the group including, but not limited to, *amd*S (acetamidase), *arg*B (ornithine carbamoyltransferase), *bar* (phosphinothricinacetyltransferase), *ble*A (phleomycin binding), *hyg*B (hygromycinphosphotransferase), *nia*D (nitrate reductase), *pyr*G (orotidine-5'-phosphate decarboxylase), *sC* (sulfate adenyltransferase), and *trp*C (anthranilate synthase), as well as equivalents from other species. Preferred for use in an *Aspergillus* and *Penicillium* cell are the *amd*S (EP 635574, WO 97/06261) and *pyr*G genes of *A*. *nidulans* or *A*. *oryzae* and the *bar* gene of *Streptomyces hygroscopicus.* More preferably an *amd*S gene is used, even more preferably an *amd*S gene from *A*. *nidulans* or *A*. *niger.* A most preferred selection marker gene is the *A.nidulans amdS* coding sequence fused to the *A.nidulans gpdA* promoter as disclosed in EP 635574, which is herein enclosed by reference. *Amd*S genes from other fungi may also be used, e.g. the ones disclosed in WO 97/06261.

The procedures used to join the above-described elements to construct an expression vector of the present invention are well-known in the art (*e.g*., Sambrook & Russell, Molecular Cloning: A Laboratory Manual, 3rd Ed., CSHL Press, 2001; Ausubel et al., Current Protocols in Molecular Biology, Wiley InterScience, 1995).

### Host Cells and Other Engineered Cells

DNA binding sites of the present invention are preferably used for engineering one of two types of cells:
(i) a first type of cell that would be highly suited for producing a desired polypeptide, the desired polypeptide being under the control of one or more DNA binding sites and
(ii) a second type of cell that would be highly suited for producing a desired polypeptide, the desired polypeptide being sensitive to protease degradation. In this type of cell, at least one DNA binding site in the host cell's genome is mutated in accordance with the second aspect of the invention such that PrtT no longer specifically recognizes the mutated, non-functional site or that PrtT no longer activates transcription.

Optionally, both types of cells additionally comprise a polynucleotide or an expression vector comprising at least one functional or mutated (*i.e*., non-functional, or enhanced) DNA binding site, which is operably linked upstream of a transcribed nucleotide sequence (*e.g*., encoding a polypeptide sensitive to protease degradation, a polypeptide under the control of a PrtT transcriptional activator, the PrtT transcriptional activator itself, or another polypeptide to be produced).

Optionally, the host cell comprises an elevated unfolded protein response (UPR) to enhance production abilities of a polypeptide of interest. UPR may be increased by techniques described in US 2004/0186070 and/or US 2001/0034045 and/or WO 01/72783. More specifically, the protein level of HAC1 and/or IRE1 and/or PTC2 has been modulated in order to obtain a host cell having an elevated UPR. Alternatively, or in combination with an elevated UPR and/or a phenotype displaying lower protease expression and/or protease secretion, the host cell displays an oxalate deficient phenotype in order to enhance the yield of production of a polypeptide of interest. An oxalate deficient phenotype may be obtained by techniques described in WO2004/070022A2.
Alternatively, or in combination with an elevated UPR and/or oxalate deficiency, the host cell displays a combination of phenotypic differences compared to the wild cell to enhance the yield of production of the polypeptide of interest. These differences may include, but are not limited to, lowered expression of glucoamylase and/or neutral alpha-amylase A and/or neutral alpha-amylase B, protease, and oxalic acid hydrolase. Said phenotypic differences displayed by the host cell may be obtained by genetic modification according to the techniques described in US2004/0191864A1.

The choice of a host cell will to a large extent depend upon the source of the nucleotide sequence encoding the desired polypeptide to be produced. Preferably, the host cell is a filamentous fungal cell as defined earlier in the description as a source where the DNA binding site may be obtained from. The host cell may also be a host cell as disclosed in WO 01/68864 or WO 00/20596. The introduction of an expression vector or other polynucleotide into a filamentous fungal cell may involve a process consisting of protoplast formation, transformation of the protoplasts, and regeneration of the cell wall in a known manner. Suitable procedures for transformation of *Aspergillus* cells are described in EP 238023 and Yelton et al. (Proc. Natl. Acad. Sci. USA 81:1470-1474, 1984). A suitable method of transforming *Fusarium* species is described by Malardier et al. (Gene 78:147-156, 1989) or in WO 96/00787. The expression vector or nucleic acid construct that can be used were already described under the corresponding sections.

### Production of a Polypeptide Sensitive to Protease Degradation

According to another embodiment, the present invention relates to a host cell with a reduced protease phenotype, which cell is a mutant of a parent cell useful for the production of a polypeptide sensitive to protease degradation, in which the parent cell comprises one or more nucleotide sequences encoding proteases, the transcription of which is activated by a PrtT transcriptional activator, and the mutant cell transcribes fewer protease genes than the parent cell when cultured under the same conditions because the protease promoters comprise one or more mutated, non-functional PrtT binding sites.

A preferred method for measurement of protease activity in a host cell is described in the example section herein for determination of the acidic endo-protease activity using bovine serum albumin (BSA) as substrate. A detailed description of this method is also described by van den Hombergh et al. (Curr. Genet. 28:299-308, 1995). Measurement of protease(s) also may be assayed using other known methods. In one such method, an aliquot of a 48 hr culture media is incubated with ³H-labeled sperm whale myoglobin at pH 4.0 and the radioactivity in the TCA-soluble fraction is measured (van Noort et al., Anal. Biochem. 198:385-390, 1991). Other methods have been described for identifying, e.g., aspartic proteinase A of *A*. *niger* (Takahashi, Meth. Enzymol. 248:146-155, 1991), endopeptidases (Morihara, Meth. Enzymol. 248:242-253, 1995), carboxypeptidases (Reminton & Breddam, Meth. Enzymol. 244:231-248, 1994), dipeptidyl peptidase (Ikehara et al., Meth. Enzymol. 244:215-227, 1994), and aminopeptidases (Little et al., Meth. Enzymol. 45:495-503, 1976). Alternatively other protease assays may be used such as the one described in WO 02/068623. Alternatively, the assay used may be Northern blotting, the use of a reporter gene under the control of a protease promoter, or a western blotting or a DNA array analysis (Eisen & Brown, Meth. Enzymol. 303:179-205, 1999) as also described herein.

A mutant cell may produce fewer proteases and less protease activity than the parental cell that is used as a reference when measured by any one of the given assays. A mutant *A.niger* may produce fewer proteases and less protease activity than the deposited *A.niger* CBS 513.88. A mutant *A. oryzae* may produce fewer proteases and less protease activity than the deposited *A. oryzae.* A mutant *P. chrysogenum* may produce fewer proteases and less protease activity than the deposited *P.chrysogenum* CBS 455.95. A mutant *A. fumigatus* may produce fewer proteases and less protease activity than *A. fumigatus* AF293 (CBS 101355).

A mutant cell may be obtained by genetic engineering using recombinant genetic manipulation techniques, submitting the filamentous fungus to mutagenesis, or both. Using genetic manipulation techniques, it is preferred to obtain a recombinant fungus: preferably by deleting a DNA binding site recognized by a PrtT transcriptional activator, more preferably the deleted DNA binding site is replaced by a non-functional variant thereof, and most preferably the deletion and replacement are made as described in EP 357127.

The mutant cell may be obtained by modification or inactivation of a DNA binding site recognized by a PrtT transcriptional activator present in the cell and necessary for expression of a downstream sequence. Expression of proteases in the mutant cell may thereby be reduced or eliminated.

Modification or inactivation of the DNA binding site of the present invention may result from subjecting the parent cell to mutagenesis and selecting for mutant cells in which the ability to express proteases has been reduced by comparison to the parental cell. The mutagenesis, which may be specific or random, may be performed, for example, by use of a suitable physical or chemical mutagenizing agent, by use of a suitable oligonucleotide, or by subjecting the DNA sequence to PCR-generated mutagenesis. Furthermore, the mutagenesis may be performed by use of any combination of these mutagenizing agents.

Examples of a physical or chemical mutagenizing agent suitable for the present purpose include gamma or ultraviolet (UV) radiation, hydroxylamine, N-methyl-N'-nitro-N- nitrosoguanidine (MNNG), O-methyl hydroxylamine, nitrous acid, ethyl methane sulphonate (EMS), sodium bisulphite, formic acid, and nucleotide analogs. When such agents are used, the mutagenesis is typically performed by incubating the parent cell to be mutagenized in the presence of the mutagenizing agent of choice under suitable conditions, and selecting for mutant cells exhibiting reduced expression of the gene.

The filamentous fungus obtained may be subsequently selected by monitoring the expression level of the desired polypeptide and/or any protease known to be under control of the PrtT transcriptional activation. Optionally, the filamentous fungus is subsequently selected by measuring the expression level of a given gene of interest to be expressed in the host cell.

The mutant cell, which has been modified or inactivated by any of the methods described above and produces fewer proteases and less protease activity than the parent cell when cultured under identical conditions as measured using the same assays as defined before, may harbor another nucleotide sequence. The mutant cell produces preferably at least 25% less, more preferably at least 50% less, even more preferably at least 75% less, and most preferably at least 95% less protease activity than the parent cell when cultured under identical conditions using the same assays as defined before. The filamentous fungus *Aspergillus niger, Aspergillus oryzae, Aspergillus fumigatus, Penicillium chrysogenum, F. oxysporum,* or *F. venenatum* mutant cell may produce fewer proteases and less protease activity than the corresponding deposited filamentous fungus cited earlier when cultured under identical conditions using the same assays as defined before.

According to an embodiment of the invention, polypeptides are consequently produced in a host cell of the present invention with a reduced protease phenotype, which cell is a mutant of a parent cell useful for the production of a polypeptide sensitive to protease degradation, in which the parent cell comprises one or more nucleotide sequences encoding proteases, the transcription of which is activated by a PrtT transcriptional activator, and the mutant cell transcribes fewer protease genes than the parent cell when cultured under the same conditions because the protease promoters comprise one or more mutated, non-functional DNA binding sites.

According to a preferred embodiment of the invention, a polypeptide is produced by a method comprising:
(a) cultivating the host cell with reduced protease phenotype in a nutrient medium, under conditions conducive to expression of the polypeptide
(b) expressing the polypeptide in said host cell, and
(c) optionally recovering the polypeptide from the nutrient medium or from the host cell.

According to another preferred embodiment of the invention, a polypeptide is produced by a method comprising:
(a) transforming the host cell with reduced protease phenotype with an expression vector, wherein the vector expresses the polypeptide,
(b) cultivating the host cell in a nutrient medium, under conditions conducive to expression of the polypeptide
(c) expressing the polypeptide in the host cell, and
(d) optionally recovering the polypeptide from the nutrient medium or from the host cell.

### Production of Other Native or Heterologous Polypeptides and Other Sequences

According to yet another embodiment, the present invention relates to methods of transcribing a downstream nucleotide sequence using a PrtT transcriptional activator in a host cell, wherein the transcribed sequence encodes a desired polypeptide or is a functional nucleic acid molecule, comprising:
(a) cultivating, in a nutrient medium, a host cell comprising (i) a promoter, (ii) a transcriptional stop signal, a (iii) translational stop signal and (iv) a non-mutated and/or a mutated, enhanced DNA binding site of the present invention, further comprising a downstream nucleotide sequence which encodes a polypeptide, wherein transcription of the downstream nucleotide sequence is activated by PrtT,
(b) expressing the polypeptide in the host cell, and
(d) optionally, recovering the polypeptide from the nutrient medium or from the host cell.
The polypeptide produced may be sensitive to protease degradation. In this case, a mutant host cell which is protease deficient will be used. The protease deficient host cell is preferably produced according to the method of the present invention. Fungi may be grown or maintained in a nutrient medium suitable for production of the desired polypeptide using methods known in the art. For example, cells may be plated on a solid substrate, shaken in a flask, cultivated in small-scale or large-scale fermentation (including continuous, batch, fedbatch, or solid-state fermentation) in laboratory or industrial fermentors in a suitable medium and under conditions allowing the polypeptide to be expressed and/or isolated. Cultivation takes place in a suitable nutrient medium comprising carbon and nitrogen sources and inorganic salts, using procedures known in the art (see, *e.g.,* Bennett & LaSure, eds., More Gene Manipulations in Fungi, Academic Press, CA, 1991). Suitable media are available from commercial suppliers or may be prepared using published compositions *(e.g.,* in catalogues of the American Type Culture Collection). If the polypeptide is secreted into the nutrient medium, the polypeptide can be recovered directly from the medium. If the polypeptide is not secreted, it can be recovered from cell lysates.

The resulting polypeptide may be isolated by methods known in the art. For example, the polypeptide may be isolated from the nutrient medium by conventional procedures including, but not limited to, centrifugation, filtration, extraction, spray drying, evaporation, or precipitation. The isolated polypeptide may then be further purified by a variety of procedures known in the art including, but not limited to, chromatography *(e.g.,* ion exchange, affinity, hydrophobic, chromatofocusing, or size exclusion), electrophoresis *(e.g.,* preparative isoelectric focusing), differential solubility (*e.g.,* acetone or ammonium sulfate precipitation), or extraction (*e.g.,* chaotrope, salt, or pH). See, *e.g.,* Janson & Ryden, eds., Protein Purification, VCH Publishers, New York, 1989.

The polypeptide may be detected using methods known in the art that are specific for the polypeptide. These detection methods may include use of specific antibodies, formation of an enzyme product, disappearance of an enzyme substrate, or SDS-PAGE. For example, an enzyme assay may be used to determine the activity of the polypeptide. Procedures for determining enzyme activity are known in the art for many enzymes.

Cells may produce at least 20% more, at least 50% more, at least 100% more, at least 200% more, or at least 300% more of the polypeptide than a corresponding parent cell when cultivated under the same conditions using one of the given assays. Preferably, the parent cell is one of the deposited strains cited earlier as host cell or as source of the DNA binding site sequence.

The polypeptide may be any polypeptide whether native or heterologous to the filamentous fungal cell. The term "heterologous polypeptide" is defined herein as a polypeptide, which is not produced by a wild-type filamentous fungal cell. The term "polypeptide" is not meant herein to refer to a specific length of the encoded produce and therefore encompasses peptides, oligopeptides and proteins. The nucleotide sequence encoding a heterologous polypeptide may be obtained from any prokaryote, eukaryote, or other source and may be a synthetic gene. The term "obtained from" as used herein in connection with a given source shall mean that the polypeptide is produced by the source or by a cell in which a gene from the source has been inserted.

The desired polypeptide may be an antibody or antigen-binding portion thereof, antigen, clotting factor, enzyme, peptide hormone or variant thereof, receptor or ligand-binding portion thereof, regulatory protein, structural protein, reporter, transport protein, intracellular protein, protein involved in a secretory process, protein involved in a folding process, chaperone, peptide amino acid transporter, glycosylation factor, or transcription factor. The polypeptide may be secreted extracellularly into culture medium.

Enzymes include oxidoreductases, transferases, hydrolases, lyases, isomerases, ligases, catalases, cellulases, chitinases, cutinase, deoxyribonuclease, dextranases, and esterases. The polypeptide may be a carbohydrase, e.g. cellulases such as endoglucanases, β-glucanases, cellobiohydrolases, and β-glucosidases, hemicellulases or pectinolytic enzymes such as xylanases, xylosidases, mannanases, galactanases, galactosidases, pectin methyl esterases, pectin lyases, pectate lyases, endo polygalacturonases, exopolygalacturonases rhamnogalacturonases, arabanases, arabinofuranosidases, arabinoxylan hydrolases, galacturonases, lyases, and amylolytic enzymes; hydrolase, isomerase, or ligase, phosphatases such as phytases, esterases such as lipases, phospholipases, galactolipases, proteolytic enzymes, carboxypeptidase, endo-protease, metallo-protease, serine-protease, amino peptidase, oxidoreductases such as oxidases, transferases, and isomerases. More preferably, the desired polypeptide is a porcine phospholipase. The polypeptide may be an amylase, carbohydrase, catalase, chitinase, cutinase, cyclodextrin glycosyltransferase, deoxyribonuclease, esterase, alpha-galactosidase, beta-galactosidase, glucoamylase, alpha-glucosidase, beta-glucosidase, haloperoxidase, proteolytic enzyme, invertase, laccase, lipase, mannosidase, mutanase, oxidase, pectinolytic enzyme, peroxidase, phospholipase, polyphenoloxidase, ribonuclease, transglutaminase, glucose oxidase, hexose oxidase, or monooxygenase.

The polypeptide may be human insulin or an analog thereof, human growth hormone, human erythropoietin, human tissue plasminogen activator (tPA), or human insulinotropin.

Alternatively the polypeptide may be an intracellular protein or enzyme such as, for example, a chaperone, protease, or transcription factor. An example of this is described by Punt et al. (Appl. Microbiol. Biotechnol. 50:447-454, 1998). This can be used for example to improve the efficiency of a host cell as protein producer if this polypeptide, such as a chaperone, protease, or transcription factor, is known to be a limiting factor in protein production.

In the methods of the present invention, the filamentous fungal cells may also be used for the recombinant production of polypeptides, which are native to the cell. The native polypeptides may be recombinantly produced by, *e.g.,* placing a gene encoding the polypeptide under the control of a different promoter to enhance expression of the polypeptide, to expedite export of a native polypeptide of interest outside the cell by use of a signal sequence, and to increase the copy number of a gene encoding the polypeptide normally produced by the cell. The present invention also encompasses, within the scope of the term "heterologous polypeptide", such recombinant production of polypeptides native to the cell, to the extent that such expression involves the use of genetic elements not endogenous to the cell, or use of endogenous sequence elements which have been manipulated to function in a manner that do not normally occur in the filamentous fungal cell. The techniques used to isolate or clone a nucleotide sequence encoding a heterologous polypeptide are known in the art and include isolation from genomic DNA, preparation from cDNA, or a combination thereof.

In the methods of the present invention, heterologous polypeptides may also include a fused or hybrid polypeptide in which another polypeptide is fused at the N-terminus or the C-terminus of the polypeptide or fragment thereof. A fused polypeptide is produced by fusing a nucleotide sequence (or a portion thereof) encoding one polypeptide to a nucleotide sequence (or a portion thereof) encoding another polypeptide.

Techniques for producing fusion polypeptides are known in the art, and include, ligating the coding sequences encoding the polypeptides so that they are in frame and expression of the fused polypeptide is under control of the same promoter (s) and terminator. The hybrid polypeptides may comprise a combination of partial or complete polypeptide sequences obtained from at least two different polypeptides wherein one or more may be heterologous to the mutant fungal cell. An isolated nucleotide sequence encoding a heterologous polypeptide of interest may be manipulated in a variety of ways to provide for expression of the polypeptide. Expression will be understood to include any step involved in the production of the polypeptide including, but not limited to, transcription, posttranscriptional modification, translation, posttranslational modification, and secretion. Manipulation of the nucleotide sequence encoding a polypeptide prior to its insertion into a vector may be desirable or necessary depending on the expression vector. The techniques for modifying nucleotide sequences utilizing cloning methods are well known in the art.

Alternatively, the downstream transcribed sequence does not encode any polypeptide but is a functional nucleic acid molecule instead. Splicing or endonucleolytic/ exonucleolytic processing of nascent transcripts may be required posttranscriptionally. The nucleic acid molecule may be an antisense or siRNA molecule.

Modification or inactivation of a host gene may be performed by established antisense techniques using a nucleotide sequence complementary to the nucleotide sequence of the gene. More specifically, expression of the gene by a filamentous fungal cell may be reduced or eliminated by introducing a nucleotide sequence complementary to the nucleotide sequence of the fungal gene, which may be transcribed in the cell and is capable of hybridizing to the mRNA produced in the cell. Under conditions allowing the complementary antisense nucleotide sequence to hybridize to the mRNA, the amount of protein translated is thus reduced or eliminated. Examples of expressing an antisense RNA is provided by Ngiam et al. (Appl. Environ. Microbiol. 66:775-782, 2000) and Zrenner et al. (Planta 190:247-252, 1993).

Modification, downregulation, or inactivation of a host gene may be obtained via RNA interference (RNAi) techniques (FEMS Microb. Lett. 237:317-324, 2004). More specifically, expression of the gene by a filamentous fungal cell may be reduced or eliminated by cloning identical sense and antisense portions of the nucleotide sequence, which expression is to be affected, behind each other with a nucleotide spacer in between, inserting into an expression vector, and introducing the expression vector into the cell where double-stranded RNA (dsRNA) may be transcribed and then processed to shorter siRNA that is able to hybridize to target mRNA. After dsRNA is transcribed, formation of small (21-23) nucleotide siRNA fragments will lead to a targeted degradation of the mRNA, which is to be affected. The elimination of the specific mRNA can be to various extents. The RNA interference techniques described in WO 2005/05672 and WO 2005/026356 may be used for modification, downregulation, or inactivation of the host gene.

### Processes for the identification of protease genes.

In a further aspect of the invention, processes for identifying protease genes are provided. One or more differentially expressed genes are detected in corresponding fungal cells either with or without a PrtT transcriptional activator *(e.g.,* deleting *prt*T or otherwise inactivating PrtT). Gene expression can be determined by methods known to the person skilled in the art. Examples of such expression analyses are aforementioned in the description and include: Northern Blot, Real Time PCR and RT-PCR. Those differentially expressed genes that encode proteases (*e.g.,* as determined by sequence similarity to known proteases, proteolytic activity of the gene product, and possession of a DNA binding site for PrtT) are identified as protease genes controlled by PrtT. Novel proteases so identified and polynucleotides encoding these novel proteases are anticipated by the present invention.

The present invention is further described by the following examples, which should not be construed as limiting the scope of the invention.

### Preferred Embodiments of the invention

1. An isolated polynucleotide comprising a double-stranded DNA binding site for a PrtT transcriptional activator, wherein at least 32 bases of a first strand of the site are identical in sequence to 5'-G/C (N)₅ C C G A/T C G G (N)₁₉ G/C-3' (SEQ ID NO:22), a second strand of the site is complementary to the first strand, and binding of PrtT to the site will activate transcription of a downstream nucleotide sequence in a host cell.
2. An isolated polynucleotide comprising a double-stranded mutated, non-functional DNA binding site, wherein at least 32 bases of a first strand of a non-mutated site are identical in sequence to 5'-G/C (N)₅ C C G A/T C G G (N)₁₉ G/C-3' (SEQ ID NO:22), a second strand of the mutated, non-functional site is complementary to the first strand, the non-mutated site is bound by a PrtT transcriptional activator and binding of PrtT to the non-mutated site will activate transcription of a downstream nucleotide sequence in a host cell, but at least one base of a first strand of the mutated, non-functional site is changed as compared to the nucleotide sequence of the non-mutated site such that PrtT no longer binds to the mutated, non-functional site or that PrtT no longer activates transcription of a downstream nucleotide sequence.
3. An isolated polynucleotide comprising a double-stranded mutated, enhanced DNA binding site, wherein at least 32 bases of a first strand of a non-mutated site are identical in sequence to 5'-G/C (N)₅ C C G A/T C G G (N)₁₉ G/C-3' (SEQ ID NO:22), a second strand of the mutated, enhanced site is complementary to the first strand, the non-mutated and mutated, enhanced sites are bound by a PrtT transcriptional activator and binding of PrtT to either the non-mutated or the mutated, enhanced site will activate transcription of a downstream nucleotide sequence in a host cell, but at least one base of a first strand of the mutated, enhanced site is changed as compared to the nucleotide sequence of the non-mutated site such that transcription of a downstream nucleotide sequence is enhanced.
4. A recombinant expression vector comprising the DNA binding site of Embodiment 1 or 3 in a promoter, a transcriptional stop signal, and a translational stop signal.
5. The vector of Embodiment 4 further comprising a downstream nucleotide sequence which encodes a polypeptide, wherein transcription of the downstream nucleotide sequence is activated by PrtT.
6. A host cell comprising the polynucleotide of Embodiments 2 or 3 or the expression vector of Embodiments 4 or 5.
7. A method of identifying a protease, wherein expression of the protease is regulated by a PrtT transcriptional activator, said method comprising:
   (a) detecting differentially expressed genes in (i) fungal cells and (ii) fungal cells with a genetic deletion of the transcriptional activator (delta *prt*T), and
   (b) identifying a differentially expressed gene that encodes a protease as a protease gene.
8. An isolated protease identified by the method of Embodiment 7.
9. An isolated polynucleotide encoding the protease of Embodiment 8.
10. A host cell, wherein at least one DNA binding site in the host cell's genome is mutated in accordance with Embodiment 2 such that PrtT can not bind to the mutated site or that PrtT can not activate transcription of a downstream nucleotide sequence.
11. A method of producing the host cell of Embodiment 10, said method comprising:
   (a) introducing the at least one mutated, non-functional DNA binding site into a promoter of the host cell by mutagenesis or recombination, and
   (b) optionally confirming reduced binding by PrtT to the mutated, non-functional site or reduced PrtT-dependent transcriptional activation in the host cell.
12. A host cell, wherein a DNA binding site in one or more of the host cell's protease gene(s) is mutated in accordance with Embodiment 2 such that PrtT can not bind to the mutated site or that PrtT can not activate transcription of the protease gene, which results in a host cell with a reduced protease phenotype.
13. A method of producing a polypeptide, said method comprising:
   (a) cultivating the host cell of Embodiment 12 in a nutrient medium, under conditions conducive to expression of the polypeptide
   (b) expressing the polypeptide in the host cell, and
   (c) optionally recovering the polypeptide from the nutrient medium or from the host cell.
14. A method of producing a polypeptide, said method comprising:
   (a) transforming the host cell of Embodiment 12 with an expression vector, wherein the vector expresses the polypeptide,
   (b) cultivating the host cell in a nutrient medium, under conditions conducive to expression of the polypeptide
   (c) expressing the polypeptide in the host cell, and
   (d) optionally recovering the polypeptide from the nutrient medium or from the host cell.
15. A method of producing a polypeptide, said method comprising:
   (a) cultivating, in a nutrient medium, a host cell comprising the vector of Embodiment 5, under conditions conducive to expression of the polypeptide encoded by the downstream nucleotide sequence comprised in said vector,
   (b) expressing the polypeptide in the host cell, and
   (d) optionally recovering the polypeptide from the nutrient medium or from the host cell.

### Examples

In the examples described herein, standard molecular cloning techniques such as isolation and purification of nucleic acids, electrophoresis of nucleic acids, enzymatic modification, cleavage and/or amplification of nucleic acids, transformation of *E.coli,* etc., were performed as described in the literature (Sambrook et al. (2000) "Molecular Cloning: a laboratory manual", third edition, Cold Spring Harbor Laboratories, Cold Spring Harbor, New York; Innis et al. (eds.) (1990) "PCR protocols, a guide to methods and applications" Academic Press, San Diego). The *Aspergillus niger* strain (CBS 513.88) used was deposited at the Centraalbureau voor Schimmelcultures Institute (CBS) under the deposit number CBS 513.88.

### Construction of a prtT knock-out of Aspergillus niger CBS 513.88

CBS 513.88 delta *prt*T*:*
This *A*. *niger* strain contains a deletion of the gene encoding the protease regulator PrtT. CBS 513.88 delta *prt*T was constructed by using the "MARKER-GENE FREE" approach as described in EP 635574. In this patent it is extensively described how to delete *gla*A specific DNA sequences in the genome of CBS 513.88. The procedure resulted in a MARKER-GENE FREE recombinant *A. niger* CBS 513.88 delta *prt*T*,* which does not contain any foreign DNA sequences.
A gene replacement vector for the *prt*T gene encoding the protease regulator was designed according to known principles and constructed according to routine cloning procedures. In essence, these vectors comprise approximately 1000-3000 bp flanking regions of a *prt*T ORF for homologous recombination at the predestined genomic locus. In addition, it contains a bi-directional amdS selection marker, in-between direct repeats. The general design of these deletion vectors is disclosed in EP 635574 and WO 98/46772, which are herein incorporated by reference.
Using oligonucleotides of SEQ ID NO:15 and SEQ ID NO: 16 as primers and genomic DNA of CBS 513.88 as template, PCR was used to amplify a 1.5 kb *prt*T downstream flanking region and introduce *Kpn*I and *Xma*I restriction sites at the ends, to allow cloning in pGBDEL (Fig. 4). This 1.5 kb *prt*T downstream flanking fragment was digested with *Kpn*I and *Xma*I and introduced in a *Kpn*I and *Xma*I digested vector pGBDEL, generating pGBDEL-PRT1.
Using oligonucleotides of SEQ ID NO: 11 and SEQ ID NO: 12 as primers and genomic DNA of CBS 513.88 as template, a 3 kb *prt*T upstream flanking region, identified as a fragment A, was amplified by PCR. Additionally, a *Bst*BI restriction site was attached to the 5'-end and an overlapping sequence of the *prt*T downstream region at the 3'-end of the fragment A. Using oligonucleotides of SEQ ID NO: 13 and SEQ ID NO:14 as primers and genomic DNA of CBS 513.88 as template, a 500 bp *prt*T downstream flanking region, identified as a fragment B, was amplified by PCR. Both resulting fragments, A and B, were fused by sequence overlap extension (SOE-PCR as described by Ho et al., Gene 77:51-59, 1989) using PCR, oligonucleotides of SEQ ID NO:11 and SEQ ID NO:14 as primers and fragments A and B; generating a 3.5 kb fragment C. This fragment C was digested with *BstB*I and *Asc*I and introduced in a Bst*B*I and *Asc*I digested vector pGBDEL-PRT1, generating pGBDEL-PRT2 (Fig. 5). The sequence of the introduced PCR fragments comprising the upstream and downstream regions of the *prt*T gene were confirmed by sequence analysis.
Linear DNA from *Bst*BI/*Xma*I-digested deletion vector pGBDEL-PRT2 was isolated and used to transform CBS 513.88. This linear DNA can integrate into the genome at the *prtT* locus, thus substituting the *prt*T coding sequence with the construct containing *amd*S (see Fig. 6). Transformants were selected on acetamide media and colony purified according to standard procedures. Growing colonies were diagnosed by PCR for integration at the *prt*T locus. Deletion of the *prt*T gene was detectable by amplification of a band, with a size specific for the pGBDEL-PRT2 insert and loss of a band specific for the wild-type *prt*T locus. Spores were plated on fluoro-acetamide media to select strains, which lost the *amd*S marker. Candidate strains were tested using Southern analysis for proper deletion of the *prt*T gene. Strains delta *prt*T were selected as representative strains with the *prt*T gene inactivated (see Fig. 6).

### Construction of a pepA knock-out of Aspergillus niger CBS 513.88

CBS 513.88 delta *pep*A:
A pepA deficient *Aspergillus niger* CBS 513.88 was generated as described by van den Hombergh et al. (Eur. J. Biochem. 247:605-613, 1997)

### Generation of a prtT knock-out of CBS 513.88 delta pepA

CBS 513.88 delta *prt*T delta *pep*A:
The method described for the construction of CBS 513.88 delta *prt*T was used to analogously construct a *prt*T knockout of CBS 513.88 delta *pep*A, to result in CBS 513.88 delta *prt*T delta *pep*A.

### A. niger shake flask fermentations

*A*. *niger* strains were precultured in 20 ml preculture medium as described in WO 99/32617. After overnight growth, 10 ml of this culture was transferred to fermentation medium 1 (FM1) with 7% glucose as described in WO 99/32617. This FM1 contains per liter: 25 g casein hydrolysate, 12.5 g yeast extract, 1 g KH₂PO₄ 2 g K₂SO₄, 0.5 g MgSO₄·7H₂O, 0.03 g ZnCl₂, 0.02 g CaCl₂, 0.01 g MnSO₄·4H₂O, 0.3 g FeSO₄·7H₂O, 10 ml pen-strep (5000 IU/ml penicillin and 5 mg/ml streptomycin), adjusted to pH 5.6 with 4N H₂SO₄. Fermentation is performed in 500 ml flasks with baffle bottoms containing 100 ml fermentation broth at 34°C and 170 rpm for the number of days indicated.

For protease induction, mycelia were harvested after culturing for 16-24 hr in FM1, washed at room temperature with Induction Medium (IM) and transferred to IM with C-source as indicated.

Induction medium (IM) contains per liter:
6 g NaNO₃ 0.5 g KCI, 1.5 g KH₂PO₄, 1.13 ml of 4M KOH, 0.5 g MgSO₄·7H₂O, 0.01% (w/v) casamino acids, 0.1% (w/v) yeast extract, 1 ml of stock trace elements (stock trace elements per liter: 22 g ZnSO₄·7H₂O, 11 g H₃BO₃, 5 g FeSO₄·7H₂O, 1.7 g CoCl_{2·}6H₂O, 1.6 g Cu₂SO₄·5H₂O, 5 g MnCl₂·4H₂O, 1.5 g Na₂MoO₄·2H₂O, 50 g EDTA, adjust the pH to 6.5 with 4M KOH, filter sterilize and store in the dark at 4°C), 10 ml of stock vitamins (stock vitamins per liter: 200 mg riboflavin, 200 mg thiamine.HCl, 200 mg nicotinamide, 100 mg pyridoxine-HCl, 20 mg panthotenic acid, 0.4 mg biotin, adjusted to pH 6 with 4M NaOH, filter sterilize and store in the dark at 4°C), and adjusted to pH 5.6, containing 1% (w/v) collagen or 2% (w/v) defatted soy flour.

### Construction of a collagen induced cDNA library

*A. niger* strain CBS 513.88 was cultivated in 100 ml of medium as described herein at 34°C and 170 rpm in an incubator shaker using a 500 ml baffled shaker flask. *A*. *niger* CBS 513.88 was precultured overnight and subsequently the mycelium was transferred to Fermentation Medium 1 (FM1 contains per liter: 25 g casein hydrolysate, 12.5 g yeast extract, 1 g KH₂PO₄, 2 g K₂SO₄, 0.5 g MgSO₄·7H₂O, 0.03 g ZnCl₂, 0.02 g CaCl₂, 0.01 g MnSO₄·4H₂O, 0.3 g FeSO₄·7H₂O, 10 ml pen-strep (5000 IU/ml penicillin and 5 mg/ml streptomycin), adjusted to pH 5.6 with 4N H₂SO₄). After 20 hr of growth, the mycelium was shifted to Induction Medium.

Mycelia harvested 18 hr, 28 hr, or 48 hr after the shift to IM containing 1% (w/v) collagen or 2% (w/v) defatted soy flour were used for RNA extractions. The RNA extractions and mRNA isolations were performed as described in detail in WO 99/32617. The construction of a cDNA expression library comprising a.o. the cDNA synthesis, the ligation of linkers and *E. coli* transformation is described as well in WO 99/32617. Linkers used for the cDNA reactions consisted of a *Hin*dIII and *Xho*I restriction sites. The resulting cDNA pools were ligated in the *Hind*III *- Xho*I digested pGBFIN-23 vector, which construction and use is described in WO 99/32617. A physical map of pGBFIN-23 can be found in Fig. 3. The ligation mixtures were used to transform DH10B electrocompetent cells (Invitrogen) resulting in the generation of over 10⁵ colonies per cDNA library obtained from both the soy flour and the collagen induced mycelium. Random sequencing of 96 clones of each of the two libraries indicated a low percentage of vectors without insert. The insert sizes for the clones sequenced were between 0.5 kb to 4.7 kb with an average of 1.7 kb. To enable an efficient screening format, the library was constructed in pools of 10³ clones. For each of these pools, glycerol stocks were made and stored for later use.

### Protease activity assays

Total acidic endoprotease activities in culture supernatants were determined as the amount of degraded bovine serum albumin (BSA). 450 µl of 1% (w/v) BSA in 0.1M NaOAc pH 4.0 was incubated with 50 µl culture supernatant at 37°C for different time intervals. At the end of the incubation period, the remainder of the BSA was precipitated with 500 µl of 10% (w/v) trichloracetic acid (TCA) and followed by incubation on ice for 10 min. The precipitate was centrifuged for 10 min at 13000 rpm in an Eppendorf centrifuge. The absorbance of the supernatant was measured at 280 nm. One unit of protease activity was defined in the Anson assay (J. Gen. Physiol. 22:79-89, 1938) as the change in absorbance units at 280 nm per hour. A more detailed description and references for this method is also described by van den Hombergh et al. (Curr. Genet. 28:299-308, 1995).

### Example 1: Detection of the PrtT-binding site

### Detection of differentially expressed genes in CBS 513.88 delta prtT

PrtT is a protease transcriptional regulator, an activator of several proteases WO 00/20596, WO 01/68864 and WO 06/040312. By the comparison of gene expression in the CBS 513.88 and CBS 513.88 delta *prt*T strains grown under the identical conditions the genes that expression is affected by the deletion of the PrtT transcriptional regulator can be detected. For the detection of differentially expressed genes several approaches, which are known in the art, can be used:
(i) Northern blot hybridization of RNA samples isolated from the CBS 513.88 and CBS 513.88 delta PrtT-1 grown under the identical conditions using specific probes for known protease-encoding genes (for the specific probes see van den Hombergh, An analysis of the proteolytic system in *Aspergillus* in order to improve protein production, Ph.D. thesis, 1996; ISBN 90-5485-545-2; van Wijk-Basten, Aminopeptidases from *Aspergillus niger,* Ph.D. thesis, 2004; ISBN 90-5808-968-1);
(ii) construction of subtraction cDNA library (methods available from companies such as Invitrogen, Genomax, and others) and identification of differentially expressed genes by sequencing of the cDNA clones; and
(iii) microarray analysis of the whole transcriptome with microarray such as the Affymetrix GeneChip® arrays.
We used a custom-made *A.niger* Affymetrix GeneChip® array to identify differentially expressed genes in CBS 513.88 and CBS 513.88 delta *prt*T strains grown under the FM1 conditions. Genes encoding proteases that showed significantly lower expression levels in the CBS 513.88 delta *prt*T strain compared to CBS 513.88 are listed in the Table 1.

### Identification of putative PrtT-binding sites

The 1.0 kb promoter region of the protease-encoding genes in Table 1 was obtained from the nucleotide sequence of the CBS 513.88 strain and contains the sequence immediately upstream from the translation initiation start point of the corresponding gene. The 1.0 kb promoter regions were analyzed using the Multiple Alignment Construction and Analysis Workbench (MACAW) program (version 2.0.5 for Macintosh). This software uses the multiple alignment algorithm (Karlin & Altschul, Proc. Natl. Acad. Sci. USA 87:2264-2268, 1990; Schuler et al., Proteins: Structure, Function, and Genetics 9:180-190, 1991; Lawrence et al., Science 262:208-214, 1993). The parameters were set up as follows: minimum pattern width: 6, maximum pattern width: 25, random seed: 12345, number of trials: 3, iterations per trial: 50.

This search resulted in the identification of a 7 nucleotide long imperfect palindrome sequence 5'-C C G A/T C G G-3' in all the protease-encoding genes experimentally detected (Table 1). Further searching upstream or downstream of the palindrome sequence in the promoters led to refinement of the sequence to a 33 nucleotide long sequence 5'-G/C (N)₅ C C G A/T C G G (N)₁₉ G/C-3' (SEQ ID NO: 22), where N = C or G or A or T (see Table 2). PrtT contains the zinc binuclear cluster Zn(II)₂-Cys₆ DNA binding domain. For a number of yeast and fungal Zn(II)₂-Cys₆ type of transcription factors the binding site has been detected and most of these sites contain two CGG half-sites aligned in inverted, everted (a sequence oriented in opposite direction, for instance target sequence is CGG, everted is CCG), or direct repeats, and separated by a fixed number of nucleotides characteristic of the transcription factor (Kim et al., Mol. Cel. Biol. 23:5208-5216, 2003; Cahuzac et al., Structure 9:827-836, 2001; Le Crom et al., Mol. Cell. Biol. 22:2642-2649, 2002 The putative binding site identified above contains the everted repeat, CCGA/TCGG, that suggest that this site is a part of the sequence for PrtT binding.

### Search of the entire A. niger genome for PrtT-binding sites

Having identified the putative PrtT-binding site in the experimentally detected set of proteases we have searched the 1.0 kb region of all predicted open reading frames (ORF's) in the genome of the CBS 513.88 for other protease-encoding genes that could be regulated via the 33 nucleotide long sequence 5'-G/C (N)₅ C C G A/T C G G (N)₁₉ G/C-3' (SEQ ID NO: 22). This search let to the identification of more than 400 ORF's. In order to select from this set of genes the possible protease encoding genes we have applied the following method:
(i) we have created a file of all Pfam domains that are identified in the MEROPS peptidase database (release 7.20, Rawlings *et al., Nucl. Acids Res.* 32:D160-D164, 2004) as the Pfam domains present in peptidases/proteases;
(ii) we used the Pfam domain list to search in the genome of the CBS 513.88 for all the ORF's that encode a protein containing a Pfam domain identified in (i).

By the overlap of the list of more than 400 ORF's that contained in the 1.0 kb promoter region the 33 nucleotide long sequence 5'-G/C (N)₅ C C G A/T C G G (N)₁₉ G/C-3' (SEQ ID NO: 22), the extended putative binding site of PrtT, and the list obtained in (ii) we have *in silico* identified additional 16 proteases that could be the potential target of the PrtT regulator. In order to confirm the identity of the gene as being a peptidase/protease, we performed the MEROPS Blast search (http://merops.sanger.ac.uk/) with the identified sequence. Out of the 16 sequences (Table 3), eight had the E-value of the MEROPS Blast search lower than e⁻²⁵ that we had set up as the cut-off value for a gene to be identified as a protease/peptidase.

Similarly, as described above for the search with the extended PrtT-binding site, we performed a search using only the imperfect palindromic sequence, 5'-C C G T/A C G G-3'. The results are described in Table 4. Additional 15 putative peptidase/protease encoding genes were identified. By applying the MEROPS Blast search E-value restriction described above, 11 genes could be specified as a peptidase/protease encoding gene.

The *in silico* search identified additionally 8 peptidase/protease-encoding genes that contain in their 1.0 kb promoter region the extended putative PrtT-binding site, 5'-G/C (N)₅ C C G A/T C G G (N)₁₉-G/C-3' (SEQ ID NO: 22), and 11 peptidase/protease-encoding genes that contain in their 1.0 kb promoter region the putative PrtT-binding site, the imperfect palindrome sequence, 5'-C C G T/A C G G-3'.

**Table 1**

| Protease encoding genes downregulated in the CBS 513.88 delta prtT strain | | |
|---|---|---|
| | | |
| | **SEQ ID NO:** | **Description** |
| 1. | 23, 24, 25 | strong similarity to protein PRO304 from patent WO200104311-A1 - Homo sapiens |
| 2. | 26, 27, 28 | aspartic proteinase aspergillopepsin I pepA - Aspergillus niger |
| 3. | 29, 30, 31 | proteinase aspergillopepsin II - Aspergillus niger |
| 4. | 32, 33, 34 | strong similarity to lysosomal pepstatin insensitive protease CLN2 - Homo sapiens |
| 5. | 35, 36, 37 | strong similarity to serine-type carboxypeptidase I cdpS - Aspergillus saitoi |
| 6. | 38, 39, 40 | similarity to putative serine peptidase - Oryza sativa |
| 7. | 41, 42, 43 | strong similarity to carboxypeptidase S1 - Penicillium janthinellum |
| 8. | 44, 45, 46 | strong similarity to hypothetical lysosomal pepstatin insensitive protease CLN2 - Canis lupus |
| 9. | 47, 48, 49 | similarity to lysosomal protease CLN2 - Rattus norvegicus |
| 10. | 50, 51, 52 | strong similarity to dipeptidyl peptidase III - Rattus norvegicus |
| 11. | 53, 54, 55 | lysine aminopeptidase apsA - Aspergillus niger |
| 12. | 56, 57, 58 | strong similarity to dipeptidyl peptidase II DPPII - Rattus norvegicus |
| 13. | 59, 60, 61 | strong similarity to hypothetical beta-lactamase XF1621 - Xylella fastidiosa |

Protease-encoding genes that are significantly downregulated in the *Aspergillus niger* CBS 513.88 delta*prt*T strain as compared to the parental *Aspergillus niger* CBS513.88 strain. The three SEQ ID NO's depicted for each gene represent: genomic DNA, cDNA and protein, respectively. The genomic DNA sequence additionally comprises a 1200bp fragment upstream of the transcription start.

**Table 2**

| **Extended binding site in the experimentally determined protease-encoding** | | | |
|---|---|---|---|
| | **SEQ ID NO:** | **Description** | **Extended binding site** |
| | | | **C/G**(N)₅CCGA/TCGG(N)₁₉**C/G** N=any nucleotide |
| 1. | 23, 24, 25 | strong similarity to protein PRO304 from patent WO200104311-A1 - Homo sapiens | **C**TACACCCGACGGAGAGCCGGGGAGAGCATCG**G** |
| 2. | 26, 27, 28 | aspartic proteinase aspergillopepsin I pepA - Aspergillus niger | **G**GAGGCCCGACGGACCCTGCGCGATCGGCGGT**G** |
| 3. | 29, 30, 31 | proteinase aspergillopepsin II - Aspergillus niger | GCTGCCCCGACGGTGAACCTTTCTGCATCCCCG GCCAAGCCGTCGGACGTCCGTCCCCCCTCTTTC |
| 4. | 32, 33, 34 | strong similarity to lysosomal pepstatin insensitive protease CLN2 - Homo sapiens | **C**GATCGCCGTCGGACTCCGGGTGGGAATCAGG**G** |
| 5. | 35, 36, 37 | strong similarity to serine-type carboxypeptidase I cdpS - Aspergillus saitoi | **G**GCTTTCCGACGGCCTCCTCTGCATCCCCTCA**C** |
| 6. | 38, 39, 40 | similarity to putative serine peptidase - Oryza sativa | **C**GCTCTCCGACGGTGCACAACAATCAATTCTG**C** |
| 7. | 41, 42, 43 | strong similarity to carboxypeptidase S1 - Penicillium janthinellum | **C**GGACTCCGTCGGCTCTGCCAGCGATCGGCAG**G** |
| 8. | 44, 45, 46 | strong similarity to hypothetical lysosomal pepstatin insensitive protease CLN2 - Canis lupus | **C**GCCCTCCGACGGCCCAAACCCACTGCAGAAT**G** |
| 9. | 47, 48, 49 | similarity to lysosomal protease CLN2 - Rattus norvegicus | **G**CAACCCCGTCGGAGTGTATACGGAGATGCGT**C** |
| 10. | 50, 51, 52 | strong similarity to dipeptidyl peptidase III - Rattus norvegicus | CTTTCCCCGACGGTGACCCGACGTAGCAGTGAC CCGCCTCCGTCGGGCCACAGCTCCGTTGGATCG |
| 11. | 53, 54, 55 | lysine aminopeptidase apsA - Aspergillus niger | **G**GACGACCGACGGAATTCCCGCGGCAAAAAGG**G** |
| 12. | 56, 57, 58 | strong similarity to dipeptidyl peptidase II DPPII - Rattus norvegicus | **G**GGAGACCGACGGATAACGCGACGATCGCCGT**C** |
| 13. | 59, 60, 61 | strong similarity to hypothetical beta- lactamase XF1621 - Xylella fastidiosa | **G**GAGCTCCGACGGGGAAACTCAGCATGTCAGC**C** |

List of the extended nucleotide sequences 5'-C/G (N)₅ C C G A/T C G G (N)₁₉ C/G-3' (SEQ ID NO:22) of putative PrtT-binding sites for the protease-encoding genes of Table 1. The imperfect palindromic nucleotide sequence defined by the MACAW program is underlined. The three SEQ ID NO's depicted for each gene represent: genomic DNA, cDNA and protein, respectively. The genomic DNA sequence additionally comprises a 1200bp fragment upstream of the transcription start.

**Table 3**

| | **Extended binding site in the protease-encoding genes detected in *silico* using palindromic CCGA/TCGG sequence** | | |
|---|---|---|---|
| | **SEQ ID NO:** | **Description** | **Extended binding site** |
| | | | **C/G**(N) ₅CCGA/TCGG(N)₁₉**C/G** N=any nucleotide |
| 14. | 62, 63, 64 | strong similarity to aspartic proteinase Yps3 - Saccharomyces cerevisiae | **G**GAGGCCCGACGGCCATCAACCGCCGAACATC**C** |
| 15. | 65, 66, 67 | strong similarity to aspergillopepsin II precursor (acid proteinase A) - Aspergillus niger | **C**AGCCACCGTCGGTCCTATCATCATCGCCCTG**G** |
| 16. | 68, 69, 70 | strong similarity to glutamate carboxypeptidase II - Rattus norvegicus | **C**CGGGCCCGTCGGATATGCGCAGGCGGTGCTG**G** |
| 17. | 71, 72, 73 | strong similarity to extracellular protease precursor Bar1 - Saccharomyces cerevisiae | **G**TAAAACCGACGGAGGTAAAACCCCGGTCATT**C** |
| 18. | 74, 75, 76 | similarity to axin-associating molecule Axam - Rattus norvegicus | **G**TGGGTCCGACGGCCATCACCCATTTCGAATT**C** |
| 19. | 77, 78, 79 | strong similarity to constitutive photomorphogenic COP9 complex chain AJH2 - Arabidopsis thaliana | **G**TGACCCCGTCGGCCCGGTAACCGCTGACTCA**G** |
| 20. | 80, 81, 82 | similarity to indole-3-acetyl-L-aspartic acid hydrolase IAA-asp - Enterobacter agglomerans | **G**GGAAGCCGTCGGCAGATGCGCCAATACGAAG**C** |
| 21. | 83, 84, 85 | strong similarity to glutamine-fructose-6-phosphate transaminase Gfa1 - Saccharomyces cerevisiae | **C**GGAGTCCGACGGAGGCACTAAAAGCGCCCCA**C** |

List of the extended nucleotide sequences 5'-C/G (N)₅ CCGA/TCGG (N)₁₉ C/G-3' (SEQ ID NO:22) of putative PrtT-binding sites detected in protease-encoding genes by *in silico* analysis of the CBS513.88 nucleotide sequence. The imperfect palindromic nucleotide sequence defined by the MACAW program is underlined. The three SEQ ID NO's depicted for each gene represent: genomic DNA, cDNA and protein, respectively. The genomic DNA sequence additionally comprises a 1200bp fragment upstream of the transcription start.

**Table 4**

| | | **Palindrome binding site in the *in silico* determined protease** | | |
|---|---|---|---|---|
| | **SEQ ID NO:** | **E value /family** | **Description** | **Palindrome binding site** |
| | | | | CCGA/TCGG |
| 22 . | 86, 87, 88 | 1.10 e-70 | similarity to carboxypeptidase D - Penicillium janthinellum | *CCGACGG* |
| | | S10 | | |
| 23 . | 89, 90, 91 | 1.1 e-111 | strong similarity to leucyl aminopeptidase Ape2 - Saccharomyces cerevisiae weak similarity to S-layer protein - Clostridium thermocellum | *CCGTCGG* |
| | | M1 | | |
| 24 . | 92, 93, 94 | 1.8 e-04 | | *CCGACGG* |
| | | S8A | | |
| 25 . | 95, 96, 97 | 1.9 e+00 | similarity to vacuolar carboxypeptidase Y Cpy - Saccharomyces cerevisiae | *CCGTCGG* |
| | | S24 | | |
| 26 . | 98, 99, 100 | 2.20 e-78 | strong similarity to ubiquitin specific protease Ubp2 - Saccharomyces cerevisiae | *CCGACGG* |
| | | C19 | | |
| 27 . | 101, 102, 103 | 5.20 e-02 | strong similarity to proteasome 19S regulatory particle subunit Rpn2 - Saccharomyces cerevisiae | CCGTCGG |
| | | M22 | | |
| 28 . | 104, 105, 106 | 6.60 e-46 | similarity to aminopeptidase P pepP - Lactococcus lactis | CCGTCGG |
| | | M24B | | |
| 29 . | 107, 108, 109 | 4.5 e-0 | similarity to hypothetical RNA export mediator like protein CAD21423.1 - Neurospora crassa | CCGACGG |
| | | 12 | | |
| 30 . | 110, 111, 112 | 1.80 e-155 | strong similarity to vacuolar aminopeptidase Ysci - Saccharomyces cerevisiae | CCGACGG |
| | | M18 | | |
| 31 . | 113, 114, 115 | 1.10-128 | similarity to lactone-specific esterase estfl - Pseudomonas fluorescens | CCGACGG |
| | | S33 | | |
| 32 . | 116, 117, 118 | 8.20 e-48 | strong similarity to prolidase - Aureobacterium esteraromaticum | CCGACGG |
| | | M38 | | |
| 33 . | 119, 120, 121 | 4.10 e-140 | strong similarity to methionyl aminopeptidase P67EIF2 - Homo sapiens | CCGACGG |
| | | M24A | | |
| 34 . | 122, 123, 124 | 1.10 e-128 | strong similarity to hypothetical protein SPAC6G10.03c - Schizosaccharomyces pombe | CCGACGG |
| | | S33 | | |
| 35 . | 125, 126, 127 | 2.10 e-193 | strong similarity to precursor of carboxypeptidase Kex1 - Saccharomyces cerevisiae | CCGACGG |
| | | S10 | | |
| 36 . | 128, 129, 130 | 6.40 e-222 | carboxypeptidase Y cpy from patent WO9609397-A1 - Aspergillus niger | *CCGACGG* |
| | | S10 | | |

List of protease-encoding genes identified *in silico* that contain the sequence 5'-C C G A/T C G G-3' in their 1.2 kb promoter regions. The imperfect palindromic nucleotide sequence defined by the MACAW program is underlined. The E-value of the Blast MEROPS search is shown in the column E-value/family together with the protease family to which the encoded protein likely belongs. The three SEQ ID NO's depicted for each gene represent: genomic DNA, cDNA and protein, respectively. The genomic DNA sequence additionally comprises a 1200bp fragment upstream of the transcription start.

### Example 2: Functionality of the PrtT-binding site

### Construction and analysis of different variants of the pepA promoter containing distinct mutations of the PrtT-binding site

A 1 kb promoter region of p*pepA* was amplified by PCR using oligonucleotides of SEQ ID NO: 1 and SEQ ID NO: 2 as primers and genomic DNA of CBS 513.88 as template, resulting in an amplification product containing SEQ ID NO: 17.

A 0.6 kb promoter region of *pep*A was amplified by PCR using oligonucleotides of SEQ ID NO: 3 and SEQ ID NO: 2 as primers and genomic DNA of CBS 513.88 as template, resulting in an amplification product containing SEQ ID NO: 18. The *pep*A coding sequence was amplified by PCR using oligonucleotides of SEQ ID NO: 5 and SEQ ID NO: 6 as primers and the cDNA library (collagen) as template, resulting in an amplification product containing SEQ ID NO: 21. PCR fragments were purified through PCR column (QIAGEN) and used in fusion PCR to obtain 1 kb *ppepA-pepA* cDNA fragment and 0.6 kb *ppepA-pepA* cDNA fragment. These were cloned and sequenced. 1 kb p*pep*A-*pep*A cDNA pCR-Blunt-Topo™ plasmid was used as a template to perform site-directed mutagenesis using a QuickChange™ site-directed mutagenesis kit (Stratagene). Table 5 gives the overview of the mutations that have been introduced in the extended 33 bp long PrtT binding sequence, 5'-G/C (N)₅ C C G A/T C G G (N)₁₉ G/C-3', of the pepA promoter. The mutations cover the imperfect palindrome sequence CCGACGG and/or the conserved terminal C/G positions. Oligonucleotides used for introducing these mutations are listed in Table 5. After mutagenesis the complete 1 kb ppepA-pepA cDNA instert in pCR-Blunt-Topo™ plasmid was re-sequenced in order to confirm the presence of the desired mutation and to exclude the presence of other mutations, possibly introduced by PCR.
One good clone for each mutant or variant was selected and used to prepare the final expression vector using *Xho*I/*Asc*I as cloning sites (Fig. 2). The obtained constructs were transformed into CBS 513.88 delta *pep*A (see Fig. 1) and in CBS 513.88 delta *prt*T delta *pep*A strain. Obtained transformants were checked by colony PCR using SEQ ID NO: 1 and SEQ ID NO: 2 (for all constructs carrying 1kb p*pep*A promoter) or using SEQ ID NO: 2 and SEQ ID NO: 3 as primers (for the construct carrying 0.6kb ppepA promoter). The integration of the constructs was targeted to the glucoamylase locus (*gla*A*,* see Fig. 2) using the flanks present in the vector. Correct transformants were grown for 10 days in 100 ml CSM/MES medium (150 g/kg maltose·H₂O, 60 g/kg Bacto soyton, 1 g/kg NaH₂PO₄ 15 g/kg (NH₄)₂SO₄, 1 g/k MgSO₄·7H₂O, 0.08 g/kg Tween-80, 0.02 g/kg Basildon, 20 g/kg MES, 1 g/kg L-arginine, pH 6.2) in 500 ml baffled shake flasks at 30°C at 250 rpm. After 10 days, the supernatant was harvested and protease activity was measured using the Anson assay (J. Gen. Physiol. 22:79-89, 1938). The results are shown in Fig. 1.

The results depicted in Fig. 1 clearly show that extracellular protease activity is strongly reduced when the palindrome CCGACGG is mutated or not present in the p*pep*A-*pep*A construct in a CBS 513.88 delta *pep*A background, indicating reduced transcriptional activity from the mutated p*pepA* promoter. The mutations in the other two conserved positions (the terminal G/C residues) in the 33bp long extended PrtT binding site affect the transcription efficiency from the pepA promoter by about 25%. Surprisingly, the combination of the mutation in the 5' end of the binding site (G to T) and insertion of one extra nucleotide in the imperfect palindrom (CCGACGG to CCGATCGG) created the pepA mutant promoter with increased transcriptional efficiency by about 40%. The sequence of the stronger *pep*A promoter is mentioned under the SEQ ID NO:139.
The dependency of the transcription of *pep*A on PrtT is further demonstrated below. When a non-modified 1.0 kb p*pep*A-*pep*A cDNA construct was transformed to CBS 513.88 delta *pep*A, a strong increase in extracellular protease activity was observed. However, when this construct was transformed into CBS 513.88 delta *prt*T delta *pep*A (data not shown) no extracellular activity was observed, indicating that *pep*A activity is highly dependent on PrtT mediated transcription. In more detail, the palindrome CCGACGG is very important for PrtT mediated transcription of *pep*A. This leads to the conclusion that CCGACGG is the PrtT-binding site in the pepA promoter. The effect of the two terminal conserved residues of the extended PrtT binding site might be in stabilization of the protein-DNA complex.

**Table 5**

| | **Extended PrtT binding site C/G** (N) ₅CCGA/TCGG (N) ₁₉**C/G** N=any nucleotide | | |
|---|---|---|---|
| **Mutant no.** | **p*pep*A PrtT binding site** GGAGGCCCGACGGACCCTGCGCGATCGGCGGTG | Forward primer | Reverse primer |
| 1. | GGAGGCCAG**A**CTGACCCTGCGCGATCGGCGGTG | SEQ ID NO. 7 | SEQ ID NO. 8 |
| 2. | GGAGGC**AA**GAC**TT**ACCCTGCGCGATCGGCGGTG | SEQ ID NO. 9 | SEQ ID NO. 10 |
| 3. | **T**GAGGCCCGA**t**CGGACCCTGCGCGATCGGCGGTG | SEQ ID NO. 131 | SEQ ID NO. 132 |
| 4. | **T**GAGGCC**A**GACGGACCCTGCGCGATCGGCGGTG | SEQ ID NO. 133 | SEQ ID NO. 134 |
| 5. | **A**GAGGCCCGACGGACCCTGCGCGATCGGCGGTG | SEQ ID NO. 135 | SEQ ID NO. 136 |
| 6. | GGAGGCCCGACGGACCCTGCGCGATCGGCGGT**A** | SEQ ID NO. 137 | SEQ ID NO. 138 |

Over-view of mutations created in the *pep*A promoter. The mutated nucleotides are depicted in bold and the PrtT binding site is underlined. The oligonucleotide primer sequences that were used to introduce these mutations are listed in the right-hand two columns.

The invention described and claimed herein is not to be limited in scope by the specific embodiments herein enclosed, since these embodiments are intended as illustrations of several aspects of the invention. Any equivalent embodiments are intended to be within the scope of this invention. Indeed, various modifications of the invention in addition to those shown and described herein will become apparent to those skilled in the art from the foregoing description. Such modifications are also intended to fall within the scope of the appended claims. In case of conflict, the present disclosure including definitions will control.

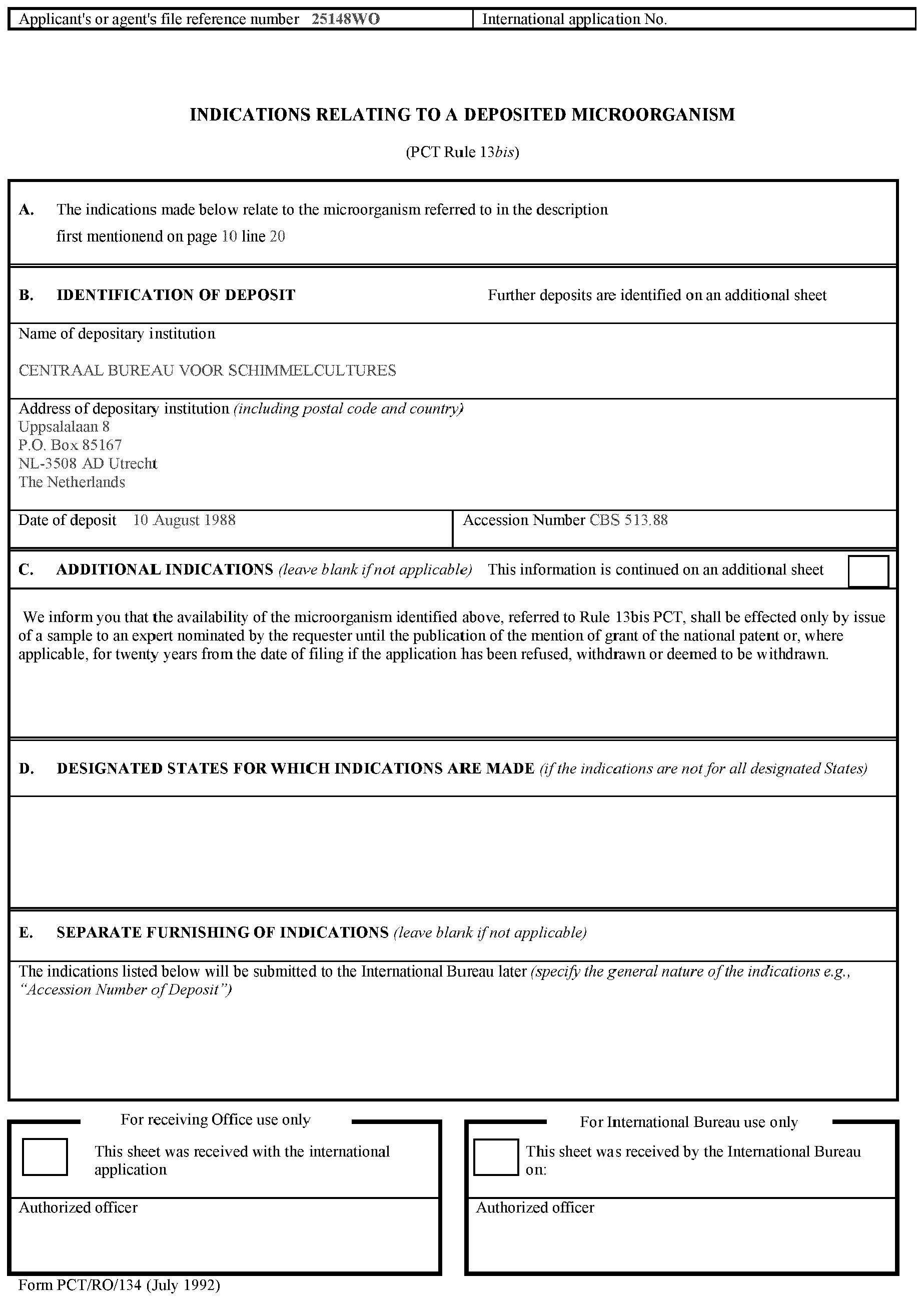

## Claims

1. An isolated polynucleotide comprising one or more double-stranded mutated, enhanced DNA binding sites having enhanced transcription of a downstream polynucleotide as compared to a non-mutated binding site , wherein a first strand of a non-mutated DNA binding site has the sequence 5'-G/C (N)₅ C C G A/T C G G (N)₁₉ G/C-3' (SEQ ID NO:22), a second strand of the mutated, enhanced site is complementary to the first strand, the non-mutated and mutated, enhanced sites are bound by a PrtT transcriptional activator and binding of PrtT to either the non-mutated or the mutated, enhanced site will activate transcription of a downstream nucleotide sequence in a host cell, wherein at least one and at most 9 non-degenerate bases of a first strand of the mutated, enhanced site are changed as compared to the nucleotide sequence of the non-mutated site such that transcription of a downstream nucleotide sequence is enhanced.

2. An isolated polynucleotide comprising one or more double-stranded mutated, non-functional DNA binding sites, wherein a first strand of a non-mutated DNA binding site has the sequence 5'-G/C (N)₅ C C G A/T C G G (N)₁₉ G/C-3' (SEQ ID NO:22), a second strand of the mutated, non-functional site is complementary to the first strand, the non-mutated site is bound by a PrtT transcriptional activator and binding of PrtT to the non-mutated site will activate transcription of a downstream nucleotide sequence in a host cell, wherein at least one base and at most 9 non-degenerate bases of a first strand of the mutated, non-functional site are changed as compared to the nucleotide sequence of the non-mutated site such that PrtT no longer binds to the mutated, non-functional site or that PrtT no longer activates transcription of a downstream nucleotide sequence.

3. An isolated polynucleotide comprising one or more double-stranded DNA binding sites for a PrtT transcriptional activator, wherein a first strand of the DNA binding site has the sequence 5'-G/C (N)₅ C C G A/T C G G (N)₁₉ G/C-3' (SEQ ID NO:22), a second strand of the site is complementary to the first strand, and binding of PrtT to the site will activate transcription of a downstream nucleotide sequence in a host cell.

4. The polynucleotide according to claim 2, wherein the non-functional prtT binding site has one or more changes in the imperfect palindrome site C C G A/T C G G.

5. A recombinant expression vector comprising the DNA binding site of Claim 1 or 3 in a promoter, a transcriptional stop signal, and a translational stop signal.

6. The vector of Claim 5 further comprising a downstream nucleotide sequence which encodes a polypeptide, wherein transcription of the downstream nucleotide sequence is activated by PrtT.

7. A host cell comprising the polynucleotide of claims 1 or 2 or the expression vector of claim 5 or 6.

8. A host cell according to claim 6, wherein at least one DNA binding site in the host cell's genome is mutated in accordance with claim 2 such that PrtT can not bind to the mutated site or that PrtT can not activate transcription of a downstream nucleotide sequence.

9. A method of producing the host cell of claim 8, said method comprising:
(a) introducing the at least one mutated, non-functional DNA binding site into a promoter of the host cell by mutagenesis or recombination, and
(b) optionally confirming reduced binding by PrtT to the mutated, non-functional site or reduced PrtT-dependent transcriptional activation in the host cell.

10. A host cell according to claim 7, wherein a DNA binding site in one or more of the host cell's protease gene(s) is mutated in accordance with claim 2 such that PrtT can not bind to the mutated site or that PrtT can not activate transcription of the protease gene, which results in a host cell with a reduced protease phenotype.

11. A host cell according to any one of claims 7-8 or 10 which is a filamentous fungal host cell, preferably a filamentous fungal host cell from a species of *Aspergillus.*

12. A method of producing a polypeptide, said method comprising:
(a) transforming the host cell of claim 10 or 11 with an expression vector, wherein the vector expresses the polypeptide,
(b) cultivating the host cell in a nutrient medium, under conditions conducive to expression of the polypeptide
(c) expressing the polypeptide in the host cell, and
(d) optionally recovering the polypeptide from the nutrient medium or from the host cell.

13. A method of producing a polypeptide, said method comprising:
(a) cultivating, in a nutrient medium, a host cell comprising the vector of claim 6, under conditions conducive to expression of the polypeptide encoded by the downstream nucleotide sequence comprised in said vector,
(b) expressing the polypeptide in the host cell, and
(d) optionally recovering the polypeptide from the nutrient medium or from the host cell.
